Europäisches Patentamt

European Patent Office

Office européen des brevets

(11) Publication number: **0 375 406**
**A2**

## (12) EUROPEAN PATENT APPLICATION

(21) Application number: 89313386.8

(22) Date of filing: 20.12.89

(51) Int. Cl.⁵: **A01K 67/027, C12N 1/21, C12N 5/10, C12N 15/00, C12P 21/08, C12N 5/12**

(30) Priority: 21.12.88 US 287713
23.06.89 US 370557

(43) Date of publication of application:
27.06.90 Bulletin 90/26

(84) Designated Contracting States:
AT BE CH DE ES FR GB GR IT LI LU NL SE

(71) Applicant: **THE TRUSTEES OF THE UNIVERSITY OF PENNSYLVANIA**
**Mellon Bank Building 133 South 36th Street**
**Suite 419**
**Philadelphia Pennsylvania 19104(US)**

(72) Inventor: **Lo, Cecilia**
**163 Jaine Lane RD-2**
**Chesterspring, Pennsylvania 19425(US)**
Inventor: **Richa, Jean**
**333 Abbey Terrace**
**Drexel Hill, Pennsylvania 19026(US)**

(74) Representative: **Woods, Geoffrey Corlett et al**
**J.A. KEMP & CO. 14 South Square Gray's Inn**
**London WC1R 5EU(GB)**

(54) Transgenic organisms and cells and methods of producing transgenic organisms and cells.

(57) Transgenic or transomic organisms and cells and methods of producing transgenic or transomic organisms and cells are disclosed. A chromosome or chromosome fragment of a donor organism is isolated and a substantial portion of the retrieved chromosome or chromosome fragment is inserted into the heritable genetic material of a recipient organism. Methods for the retrieval, manipulation, injection and subsequent insertion of large chromosome fragments into the heritable genetic material of recipient cells are disclosed.

EP 0 375 406 A2

# TRANSGENIC ORGANISMS AND CELLS, AND METHODS OF PRODUCING TRANSGENIC ORGANISMS AND CELLS

The present invention relates to one-celled and multicellular transgenic organisms (animals, plants and microbes) and to methods for producing these organisms.

The transfer of genetic material from one cell to another has recently received much attention for such uses as producing transgenic animals which express foreign genetic traits. Gene transfer has been reviewed by Scangos et al, Adv. Gen., (1987) 24:285, and Houseman and Nelson, "Use of Metaphase-Chromosome Transfer for Mammalian Gene Mapping", in Kucherlapati, Ed., Gene Transfer, pp 95-115, Plenum Press, New York, 1986.

U.S. patent No. 4,736,866 to Leder and Stewart discloses transgenic mammals having DNA relating to foreign oncogenes incorporated into their genomes for use in testing the carcinogenic effects of chemicals upon mammals. Part of the method used to make these transgenic mammals is disclosed in U.S. Patent No. 4,873,191 to Wagner and Hoppe, and in Proc. Nat. Acad. Sci. (USA), (1981) 78:5016.

Transfer of foreign genes to other species also has applications in transferring disease resistance to susceptible species and production of commercially useful compounds by plants, animals, and microbe, and by cultured plant, animal, and microbial cells. Although several techniques exist for transferring DNA from one cell to another, these methods suffer from lack of predictable and/or efficient incorporation of the foreign DNA into the recipient genome and severe limitations on the size of the genetic DNA which can be transferred. These are some of the disadvantages of the technology disclosed, e.g., in U.S. Patent No. 4,873,191.

Microinjection of DNA directly into the pronucleus of fertilized eggs is a widely used method for transferring DNA from one cell to another. In this technique, fragments of exogenous DNA are injected into the pronucleus of fertilized oocytes, such as mouse oocytes, using a very small pipette. The DNA injected is generally recombinant DNA incorporated within a plasmid cloning vector.

Prior to injection, the plasmids containing the exogenous DNA are often cut with restriction endonuclease enzymes to linearize them or to excise a recombinant insert. DNA is then either injected with the vector DNA, or the insert containing the DNA fragment is isolated and then injected. One to several hundred copies of the exogenous DNA are usually injected into each pronucleus. Subsequent to DNA injection, the embryos are transferred into a pseudopregnant female for development to term.

Typically, integration of the exogenous DNA occurs at one site in the recipient cell's genome, but some transgenic organisms, such as transgenic mice may contain multiple integration sites. The mechanism(s) of DNA integration is still not really understood. The exogenous DNA in such transgenic animals can be expressed and usually is stably transmitted to subsequent generations through the germ line.

U.S. Patent Nos. 4,736,866 and 4,873,191 disclose transgenic non-human mammals which were produced by this method. In U.S. Patent No. 4,736,866 plasmids containing the mouse myc gene or the S107 plasmacytoma myc gene were injected into male pronuclei of fertilized one-cell mouse eggs. The myc gene is known to be activatable to increase the probability of the development of neoplasms, particularly malignant tumors, in the animal.

Approximately 500 copies of linearized plasmid per pronucleus were injected. The injected eggs were then transferred to pseudopregnant females to develop to term. Mice containing the myc oncogene can be used to test a material suspected of being a carcinogen by exposing them to the suspected carcinogen and determining neoplastic growth as an indicator of carcinogenicity.

Although this method of injecting recombinant DNA into the pronuclei of fertilized eggs is effective, it is only efficient in the transfer of relatively small pieces of DNA, usually less than 100 kilobases (kbs) of contiguous DNA. This size limitation probably results from nucleolytic degradation of the injected DNA by the presence of endogenous nucleases in the recipient cell. In addition, breakage due to mechanical shear also limits the feasibility of expelling very large DNA molecules through the bore of a small pipette tip.

Similarly, DNA introduced into cells via calcium phosphate or polycation precipitated DNA also results in the incorporation of relatively small contiguous pieces of DNA, having a size normally less than 100 kilobases (kbs). With this different approach, again it is likely that nucleolytic degradation of the DNA by endogeneous nucleases limit the size of the DNA which can be inserted with this technology.

The drawbacks of the prior DNA microinjection and calcium phosphate/polycation DNA precipitation methods are especially obvious when it is desired to transfer genetic traits encoded by a very large gene, such as the dystrophin gene of the Duchenne muscular dystrophy syndrome, or the Rb gene of retinoblastoma, or those genetic traits that are encoded by a large complex of many genes, such as the human immunoglobulin complex and the histocompatability locus. The human immunoglobulin complex is comprised of genes that are derived from the joining of non-contiguous segments of DNA that are separated by great genetic distances.

Hence to transfer into a recipient cell or organism the ability to express the entire repertoire of human immunoglobulin complex, hundreds of thousand of kilobases of DNA encoding the whole complex must be transferred. Heretofore this has not been possible because of the size limitations inherent in the previously known DNA transfer methods.

In addition, with DNA injection methods, the desired gene must first be cloned before such procedures can be used to make transgenic organisms. The need for cloned DNA is a serious drawback of the microinjection technique when it is desired to transfer genetic traits for which no recombinant clones are available or otherwise for which no molecular details are known.

Although it is presently known that many traits have a genetic component, the genes encoding many of these traits have not yet been identified. The inability to transfer these traits has slowed the study and understanding of these traits.

For example, in plants such as tomatoes, heritable traits governing stature, disease resistance, and yield have been identified, and chromosome location for some of the genes governing these traits are known. However, since no molecular details are available, it has not been possible to obtain recombinant clones for genes governing these traits, without which these heritable traits cannot be transferred using available gene transfer methods.

While larger DNA inserts can be obtained with transfections mediated by calcium phosphate or polycationic precipitated chromosomes, this approach yields much lower transformation efficiencies, usually with one transformant obtained in $10^5$ to $10^7$ recipients as compared with 5 to 30 transformants obtaining from 100 recipients after DNA microinjection. Hence the transfection of chromosomes is not a practical method for introducing exogenous DNA into embryos or cells, particularly when these are available only in small numbers.

Even for cells and embryos available in large numbers, given the random nature of exogenous DNA insertion and the much lower transformation efficiency, a selectable marker in the chromosome region of interest must exist to allow the selective survival and growth of only those cells that have incorporated the appropriate exogenous chromosome or chromosome segment. This requirement for the co-localization of a selectable marker is highly impractical, and has effectively limited the application of this method to just a few chromosome regions flanked by selectable markers, such as a portion of the X chromosome containing the HGPRT locus.

Although recently the nonselective injection of total plant chromosomes extracted in bulk from suspension cultured cells of Petunia alpicola into Petunia hybrida protoplasts was described (Griesbach, Plant Science, (1987) 50:69-77), this technology does not overcome the problem of the random and nonspecific nature of exogenous DNA incorporation. It does not permit the selective isolation of a particular chromosome or the selective injection of a particular chromosome. In reality, the practical outcome of this approach is not very different from that achieved using calcium phosphate or other polycationic precipitation methods for transfecting chromosomes into tissue culture cells.

A serious drawback with either the chromosome transfection or injection methods is that the incorporation of foreign genetic information is a random process. Thus for example, with the injection of Petunia alpicola chromosomes into Petunia hybrida, or the transfection of calcium phosphate precipitated chromosomes into tissue culture cells, all chromosomes have an equal probability of being introduced and inserted into the recipient cell. However, given the genome complexity of any given organism, the likelihood that a desired trait(s) or gene(s) would be incorporated into a recipient cell is highly unlikely in the absence of a selection scheme.

It is also interesting to note that although the previously published petunia study claims that multiple enzymes in the flavonoid biosynthetic pathway were transferred from the Petunia alpicola into Petunia hybrida, (1) there was no direct evidence to document the successful transfer of DNA from the donor into the recipient, and (2) this outcome is highly improbable given that such enzymes are likely encoded by multiple gene loci, and would require the simultaneous incorporation of DNA from several different chromosomes, a highly unlikely event given the known efficiency of exogenous DNA integration.

In summary, although DNA microinjection is effective in transferring foreign DNA from one cell to another, this method is limited because it is not very useful for transferring pieces of DNA much larger than about 100 kilobases. This method is also inadequate for transferring genetic traits for which no recombinant clones are available, or for which no molecular genetic details are otherwise known.

Using the calcium phosphate or polycationic chromosome precipitation methods, it may be possible to transfer larger pieces of DNA and genomic DNA that are uncloned. However, the rate of incorporation of the transfected DNA using this latter technique is very low and the insertion of foreign genetic information is random. Hence the calcium phosphate precipitation method is impractical for use in situations where the number of recipient cells is limited and when the chromosome region to be transfered does not contain a selectable marker.

The injection of unselected whole chromosome mixtures suffers the salient disadvantage that it does not permit selection of the chromosome or chromosome region of interest which is to be integrated. With this latter approach a selectable marker is needed to identify the appropriate transformant. This severely limits the usefulness of such a procedure.-

There is thus a need for a method permitting the selective retrieval and introduction of particular region- (s) of specific chromosome(s) from one organism (cell) into another organism (cell), thereby ensuring that the transformants generated will have a reasonable probability of containing the desired genetic information.

There is thus also a need for a method permitting the transfer of large molecules of DNA (e.g. a chromosome or fragment thereof) from a donor organism into a recipient organism, and there is a particular need for such a method which would not suffer the disadvantages suffered by existing techniques as outlined above.

Accordingly the objects of the present invention include providing methods for transferring at least a chromosome or chromosome fragment to a recipient cell, methods for producing one-celled and multicellular transgenic organisms, and the transgenic organisms and single cells produced thereby.

In a general illustrative embodiment, the inventors have discovered that these objects, and others which are apparent from the description of the invention given hereinbelow, are satisfied by a novel method for transferring a chromosome or chromosome fragment from a donor organism to a recipient organism. This method comprises:

(a) isolating a chromosome or chromosome fragment from a donor organism; and

(b) inserting a substantial portion of the chromosome or chromosome fragment of the donor organism into the heritable genetic material of a recipient organism.

In other general illustrative embodiments, the present invention also provides transgenic cells containing at least one exogenous chromosome(s) or at least one exogenous chromosome fragment(s) introduced into the cell, or antecedent generation of the cell, by the method of the invention. The transgenic cells may be prokaryotic or eukaryotic cells. The transgenic cells may be single cell organisms or single cells belonging to a multicelled organism.

In still other general illustrative embodiments, the invention also provides transgenic organisms, including animals and plants, whose germ cells and somatic cells contain at least one exogenous chromosome(s) or chromosome exogenous chromosome fragment(s). These exogenous chromosome(s) or chromosome fragment(s) are obtained, independently, either from an animal or a plant, and introduced into an embryonic stage of the animal or plant, or an ancestor of the animal or plant, by the method of the invention. The transgenic organisms provided by the invention include single-celled and multicellular organisms.

The member of chromosome or chromosome fragment which can be inserted in the recipient cell in accordance with the present invention is from one to a number corresponding about to the number of chromosomes found in the native recipient cell.

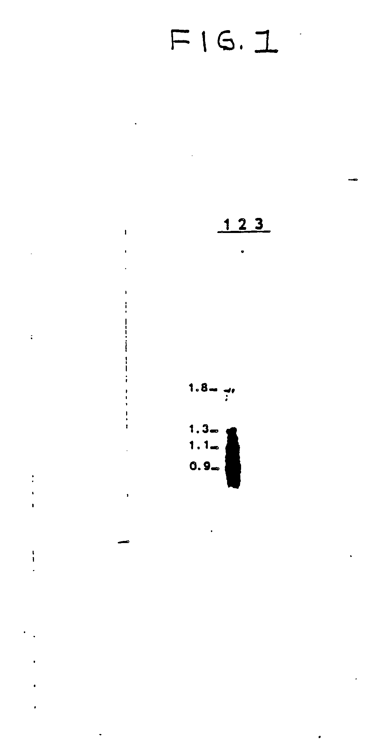

FIGURE 1 represents a Southern blot hybridization analysis. DNA (2.5 μg) extracted from mouse tail tissue was separated by agarose gel electrophoresis; blotted onto nitrocellulose and hybridized with nick-translated DNA from pBLUR8, a plasmid containing the human interspersed Alu repetitive DNA. Molecular weights are indicated in kilobases. Lane 1, MboI digested DNA from a mouse developed from an oocyte microinjected with a non-centromeric human chromosome fragment (herein "E mouse"); Lane 2, non-digested DNA from E mouse; and Lane 3, MboI digested DNA from a mouse developed from a normal, non-injected oocyte. DNA in lanes 1 and 2 both exhibited the presence of human DNA as indicated by the observation of hybridized bands. In comparison, the DNA from any uninjected normal mouse exhibited no hybridization to the pBLUR8 probe.

The term "isolating" is used in this text, in step (a) of the method, to distinguish the present method from the process of natural sexual reproduction in animals and plants. This term reflects the fact that in the present method, in the isolating step, the chromosome or chromosome fragment from the donor organism is removed from a cell of the donor organism.

To more clearly distinguish from transgenic cells and organisms available from prior art methods, the transgenic cells and organisms of the present invention which have transferred, to them or to an ancestor of such organisms and cells, at least one chromosome or chromosome fragment, are also referred to in this text as "transomic" cells or organisms. As used herein, the term "transomic" organism or cell is intended to encompass a transgenic organism or cell having had transferred to said organism or cell (or to an ancestor thereof) a chromosome or chromosome fragment according to the method of the present invention.

4

The term "organism" used herein encompasses both one-celled and multicellular organisms.

The method of the present invention for transferring a chromosome or chromosome fragment is advantageous over prior art DNA microinjection methods because these prior art methods are not useful for transferring DNA sequences larger than about 100 kilobases, e.g., the methods of U.S. Patent Nos. 4,736,866 and 4,873,191. By contrast, the present method is particularly suited for transferring DNA sequences having at least about 10 to 30 megabases. This permits transfer of genetic traits encoded by very large genes or a complex of genes. The term "bases", as in, e.g., kilobases, megabases, etc. is used in this text to indicate bases of nucleic acids.

Further, unlike the prior art calcium phosphate and polycationic-mediated chromosome precipitation methods which permit only a low rate of incorporation of the foreign DNA, the present method produces high rates of incorporation of the transferred foreign DNA. It is thus useful in instances where the number of recipient cells is limited.

Moreover, unlike the previously described chromosome injection into plant protoplasts, the present method enables the intelligent retrieval and intelligent transfer of selected chromosome(s) and chromosome fragment(s) containing identified gene(s) as well as selected genetic material containing identified trait(s) whose underlying genetic components are unknown. The present invention, in contrast to existing techniques which use a shotgun approach to gene transfer, permits the intelligent selection and introduction of genetic information into a recipient organism. The term "selected" is used in this text to reflect this intelligent retrieval and transfer provided by the invention.

The present invention also significantly differs from the method for the transfer of genetic material in making transgenic mammals described in U.S. Patent No. 4,873,191. One very important difference is that the genetic material microinjected in fertilized oocytes (zygotes) for making transgenic animals in U.S. Patent No. 4,873,191 consists of naked recombinant DNA molecules; that is, a simple, relatively short, polydeoxyribonucleic acid molecule. In contrast, in the present invention, the genetic material used (transferred) corresponds to chromosomes, that is very large DNA molecules packaged with proteins and exists in a compact state.

The chromosomes and chromosome fragments used in the invention are characterized by being associated with protein which corresponds to that found in the native chromosome, or which is at least a synthetic substantial equivalent thereof. It should be recognized that some minor modification of the associated proteins is possible, e.g., some of the proteins may be removed or enzymatically modified (e.g., due to manipulation steps or it may be synthetically modified, etc.). But a sufficient amount of this protein should remain to provide the advantages discussed below.

This protein association serves several important functions in facilitating the integration of the exogenous genetic material. First, the compact packaging of the large DNA molecules in chromosomes allows very large segments of DNA to be introduced into the recipient cell without breakage caused by mechanical shear. Second, the associated proteins protect the DNA from nucleolytic degradation by endogenous nucleases, and hence further facilitates the incorporation of large contiguous segments of DNA without nuclease-induced breaks.

The protection from both mechanical shear and nuclease-induced breakages makes it possible for DNA molecules of greater than 10 to 30 megabases (Mbs) to be incorporated into the recipient cell genome using chromosome injection methods as described in this text. In contrast, with the microinjection of naked DNA as described in U.S. Patent No. 4,873,191, the size of the contiguous DNA fragment integrated usually is no greater than 50 to 100 kbs.

A third significant advantage with the present method for the introduction of foreign genetic material via chromosome or chromosome fragments is the much higher transformation efficiency which is achieved. This higher transformation efficiency is likely due to the protection of the exogenous DNA from nucleolytic degradation. It is also possible that the packaging of DNA with proteins in chromosomes further facilitates DNA insertion by other means, such as in promoting interaction of the exogenius DNA with enzymes which mediate DNA recombination and repair. The packaging of DNA in chromosomes may also facilitate DNA insertion by enhancing and stabilizing base pairing between the exogenous and endogenous DNA.

Thus, using the present techniques, the inventors have observed successful incorporation of human DNA using microinjection of a single human chromosome fragment, while injection of naked DNA usually requires the introduction of many hundreds of copies of the exogenous DNA molecules to achieve successful incorporation of the foreign DNA.

Another important distinction of the present invention compared to the disclosure of U.S. Patent No. 4,873,191 is that it provides methods for selecting and retrieving specific segments of DNA, greater than a few megabases, from native chromosomes for insertion into a recipient cell for DNA transformation. Similarly, the present invention also differs significantly from the published study of Griesbach, supra, in which unselected whole chromosome extracts were indiscriminantly injected into plant cells.

A further significant difference of the present invention compared to the disclosure of U.S. Patent No. 4,873,191 is that it provides methods for DNA transformation by inserting exogenous genetic material into the pronucleus, nucleus, as well as cytoplasm of the recipient cell. In comparison, in U.S. Patent No. 4,873,191, the exogenous DNA mustt be introduced into the pronucleus of a mammalian zygote (fertilized oocyte). The feasibility of introducing exogenous genetic material into the cell cytoplasm for achieving DNA transfromation using methods of the present invention is a direct result of the fact that chromosome proteins shield the exogenous DNA from nucleases in the cell cytoplasm.

Also, with the present invention, the use of pregnant females has been found to be preferred over the use of pseudopregnant females for carrying injected embryos to term. In U.S. Patent No. 4,873,191, only pseudopregnant females are used for carrying the injected embryos to term. The use of pregnant females is preferred in the present invention as it provides notably greater efficiency of development to term.

The present invention provides transomic cells and organisms in which a substantial portion of (a) chromosome(s) or chromosome fragment(s) isolated from (a) donor cell(s) or organism(s) is (are) inserted into the heritable genetic material of a recipient cell or organism. The donor cell or organism may be, independently of the recipient cell or organism, a microbe, a plant or an animal, or a cell of microbial, plant or animal origin. The recipient cell or organism may also be, independently of the donor cell or organism, be a microbe, a plant or an animal, or a cell derived from a microbe, a plant, or an animal.

The present invention thus provides transomic cells and organisms which independently of whether the recipient cell or organism is a microbe, a plant or an animal, has had transferred to it (or to a ancestor thereof) (a) chromosome(s) or chromosome fragment(s) itself (themselves) obtained from (a) donor cell(s) or organism(s) which may independently be (a) microbe(s), (a) plant(s) or (an) animal(s).

In one embodiment of the present invention, the donor cell or organism and the recipient cell or organism are two separate individual cell or organisms. Independently of each other, they may be of the same or different species, same or different genus, etc. Moreover, the donor cell or organism may be, independently of the recipient cell or organism, a microbe, a plant cell, or an animal cell. Likewise, independently of the donor cell or organism, the recipient cell may be a microbe, a plant cell, or an animal cell.

In another embodiment, the donor cell and the recipient cell both correspond to the same multicellular organism. In this embodiment, two cells corresponding to the same single organism can be taken from the same or different tissues of the organism, with one srving as donor, and one serving as recipient.

The method of the invention is particularly valuable for generating transomic cells and organisms that are useful for a number of different applications, including, but not limited to:

(1) transomic cells and organisms (microbes, plants, animals) which produce products of commercial and/or medical value such as immunoglobulins or antibodies or fragments thereof, hormones, growth factors, clotting factors, enzymes such as plasminogen activators, etc.;

(2) transomic improved strains of organisms (microbes, plants, animals) which possess desirable traits such as drought/pest resistance, herbicide resistance, disease resistance, faster growth and/or reproductive rates, etc.;

(3) transomic animals and cells that serve as animal modeling systems, including human diseases and other pathological conditions, which are useful for developing drug therapies, diagnostic reagents and/or protocols and other patient management schemes, for example, such as, in Huntington's disease, adult polycystic kidney disease, Alzheimer's disease, Down's syndrome, cancer, autoimmune diseases, cardiovascular diseases, infectious diseases such as AIDS, hepatitis, or other pathological conditions of known and unknown origins, etc.;

(4) transomic cells and organisms that can serve as a chromosome library to facilitate genetic linkage mapping, and whole genome sequencing. Examples of such applications of the present technology would include the construction of human chromosome libraries for the human genome sequencing project, and the construction of plant and animal chromosome libraries for genetic linkage mapping to facilitate the breeding of valuable traits in plants and animals of commercial and/or agricultural importance; and

(5) transomic cells and organisms in which the incorporation of the injected exogenous chromosome fragment or the replacement of specific segments of chromosome(s) by the injected exogenous chromosome or chromosome fragment results in the alleviation or correction of a pathological state or the desired modification of a selected trait or traits.

The present invention also provides transomic cells or organisms containing multiple identical or different exogenous chromosome or chromsome fragments, and methods for making these. This includes transomic cells or organisms with any combination of exogenous chromosome and chromosome fragment. Such chromosomes or chromosome fragments can be derived from one or several different species of organisms, and if from one species, such chromosome fragments can be derived from the same or different cells of the same or different individuals.

The transomic cells of the invention may be prokaryotic or eukaryotic cells, such as microbial and animal or plant cells. The invention further provides transomic animals and plants whose germ cells and somatic cells contain an exogenous chromosome or an exogenous chromosome fragment introduced into a fertilized or unfertilized oocyte, sperm, germ cell, a somatic cell of the animal or plant, an embryonic stem cell of the animal or plant, or an ancestor of the animal or plant by the methods of the invention. In one embodiment, the transgenic animals of the invention are preferably non-human mammals, more preferably mice.

The methods of the invention for transferring a chromosome or chromosome fragment from a donor cell or organism to a recipient cell or organism comprise the steps of isolating a chromosome or chromosome fragment of the donor cell or organism and inserting a substantial portion of the nucleic acids, that is greater than 20% of the original number of nucleic acid bases, of the chromosome or chromosome fragment of the donor cell or organism into the heritable genetic material of the recipient cell or organism. The methods of the invention make possible the transfer of an identified gene or genes, as well as the transfer of genetic material containing an uncharacterized gene or genes.

The isolating step of the invention may further comprise the steps of (1) preparing the chromosome or chromosomes of the donor cell or organism for retrieval; (2) selecting the chromosome or chromosome segment of interest, (3) retrieving the chromosome or chromosome fragment; and (4) manipulating the retrieved chromosome or chromosome fragment into a pipette or other suitable device (vide infra).

The preparing step comprises any combination of steps which results in chromosomes suitable for the subsequent retrieval step. The selecting step may comprise any combination of steps which allow the identification of the chromosome(s) or chromosome segment(s) to be retrieved. The chromosome identification may be carried out by examining chromosome morphology, by conventional chromosome banding methods used in karyotyping, or alternative methods such as in situ hybridization analysis, or selective antibody staining of chromosome specific antigens.

If a chromosome fragment is to be transferred, the retrieving step may comprise the steps of cutting a chromosome fragment from the chromosome of the donor organism and displacing the chromosome fragment relative to the other chromosomes of the donor organism. If a whole chromosome is to be transferred, the retrieving step may comprise displacing the entire chromosome relative to the other chromosomes of the donor organism.

The manipulating step may further comprise contacting the retrieved chromosome or chromosome fragment with a mobilizing solution and aspirating the retrieved chromosome or chromosome fragment and the mobilizing solution into a pipette or other suitable device (vide infra).

Among other approaches which can be used in this manipulating step is the utilization of a mobilizing solution containing lipid components that allow the encapsulation of the retrieved chromosome or chromosome fragment within a lipid vesicle. Alternatively, the retrieved chromosome or chromosome fragment can be adsorbed onto a particle for cell insertion via a microprojectile gun (Klein et al, Nature (1987) 327:70-71).

In place of a pipette, the chromosome or chromosome fragment can be aspirated into a syringe, a microelectrode, or other suitable devices with positive displacement capability.

The manipulating step may further comprise the step of diluting the mobilizing solution within the pipette, or other suitable device, with a physiologically acceptable liquid. This step is preferably performed when the mobilizing solution contains constituents that may be harmful to the recipient organism.

Alternatively, the mobilizing solution may be omitted and the retrieved chromosome or chromosome fragment may be contacted with the physiologically acceptable solution. The chromosome or chromosome fragment and the physiologically acceptable solution is then aspirated into the pipette, or other suitable device, or the chromosome or chromosome fragment can be further processed using the alternative schemes outlined above for the manipulating step.

All of the steps involved in the chromosome/chromosome fragment isolation step can be carried out at any suitable temperature. For the step of chromosome preparation, if the chromosomes are to be prepared by standard methods, it is preferred that the procedure be carried out at recommended temperature conditions (see for example MacGregor and Varley, "Working with Animal Chromosomes," John Wiley and Sons (1983)). For the selecting, retrieving and manipulating steps, a wide range of temperature would be suitable. This wide range of temperatures is preferably between 10 and 25° C, most preferably at about room temperature.

For the step of chromosome preparation, high humidity can be detrimental and it is generally best performed under conditions of low or minimal humidity (i.e. ≦ 50% relative humidity). However, the selecting, retrieving and manipulating steps may all be carried out over a wide range of ambient humidity.

The chromosome or chromosomes of the donor organism may be visualized before the retrieving step. The visualizing step preferably comprises staining the chromosome or chromosomes of the donor organism with a stain substantially non-toxic to the recipient organism. Alternatively, the chromosome or chromosomes of the donor organism may be visualized by various optical methods such as phase contrast illumination, Hoffman modulation, Nomarsky optics, or differential interference contrast optics.

In a preferred embodiment, the visualization step can be used in the selecting step to assist in chromosome identification. The identity of some chromosomes can be determined by their overall shape and relative arm lengths. In cases of ambiguity due to the presence of multiple chromosomes of similar morphology, various methods known in this art including chromosome banding analysis, in situ hybridization analysis or antibody staining of chromosome specific antigens can be used to facilitate chromosome identification.

The inserting step of the methods of the invention may further comprise using a pipette, or any of the other devices having displacement capability discussed supra, containing the chromosome or chromosome fragment in the step of injecting a chromosome or chromosome fragment into a recipient cell or organism under conditions which allow the chromosome or chromosome fragment to be incorporated into the heritable genetic material of the recipient cell or organism.

Aside from the use of injection, via pipette, for the inserting step, other methods of insertion may be used. Some examples of other suitable methods include the use of lipid vesicles to mediate membrane fusion for inserting exogenous genetic material into recipient cells, or the use of a microprojectile gun for inserting particles adsorbed with chromosomes or chromosomes fragments into a recipient cell.

The inserting step may be carried out with insertion of multiple chromosomes or chromosome fragments. This may include chromosomes or chromosome fragments obtained from the same or different cells of one species, or from the same or different cells of different species, and also may include artificial chromosomes (discussed below), or any combination of chromosomes, chromosome fragments, or artificial chromosomes.

The inserting step is carried out in a fashion which results in the exogenous genetic material being introduced into the nucleus or pronucleus of the recipient cell. Alternatively, mitotic or meiotic cells with no nuclei are also suitable recipients in the inserting step. In the latter instance, the exogenous genetic material becomes incorporated into the recipient cell nucleus as the nuclear membrane reforms upon the end of mitosis or meiosis.

The integration of the exogenous genetic material is mediated by the endogenous recombination and DNA repair enzymes normally present in the recipient cell. The present method makes possible that a substantial portion of all of the chromosome or chromosome fragment is incorporated into the heritable genetic material of the recipient cell. This incorporation level is sufficient to provide the desired trait to be observed in the transomic organism produced. Thus generally this level of incorporation may advantageously include at least more than about 20% of the exogenous genetic material transferred.

However, some of the chromosome material may be lost prior to incorporation into the recipient cell genome. This can result if, for example, a sufficient amount of the proteins associated with the chromosome or chromosome fragment are lost, thereby permitting some limited nucleolytic degradation of the exposed DNA domains. Such partial loss of the associated protein may occur for a variety of reasons, such as degradation due to proteolysis, or mechanical perturbations, etc.

Nonetheless, the incorporation of the exogenous chromosome or chromosome fragments, artificial or naturally occurring, into the cell, results in the integration of the exogenous DNA into the endogenous chromosomes of the cell. However, it is also possible that the exogenous genetic material may exist as episomal elements in the genome of the recipient organism; that is without insertion into the chromosomes of the recipient cell or organism.

In such cases, the exogenous chromosomal material may persist as a separate chromosome, with the ability to partition through successive rounds of cell division. This is most likely to occur when various DNA elements such as centromeric, telomeric, and replication origin sequences are included in the exogenous genetic material, either naturally or through synthetic design. However, it is also possible that such sequence elements can be incorporated into the exogenous genetic material by acquisition from the genetic information present in the recipient cell genome.

In the event that multiple chromosome fragments or chromosomes are introduced in the inserting step, then one or more of these exogenous protein associated DNA molecules may be incorporated into the genome of the recipient cell, and their incorporation will be mediated by mechanisms similar to that for a single chromsome or chromosome fragment. Note that in the presence of multiple exogenous chromosomes or chromosome fragments, physical association of these independent macromolecules in the recipient cell may result in their covalent linkage via the action of endogenous DNA recombination and repair enzymes. In such instances, the multiple exogenous chromosome or chromosome fragments can become cointegrated in the recipient cell genome.

The source of the chromosome or chromosome fragment used in the present invention may be of animal, plant or microbial origin. The chromosomes and chromosome fragments used in the invention may also be synthetic modifications of naturally occuring chromosomes and chromosome fragments, or totally new, synthetically produced, large DNA molecules having a protective proteinacecus coat as described supra.

Such synthetically modified naturally occurring chromosomes or totally new synthetically produced protein associated DNA molecules may be referred to as artificial chromosomes in this text where such distinction is made.

Such artificial chromosomes may be generated in a variety of ways. For example, multiple naturally occurring chromosome or chromosome fragments may be linked together prior to insertion. This may involve covalent ligation, or noncovalent association of the chromosomes or chromosome fragments. The naturally occurring chromosome or chromosome fragments might also be modified by sequence deletions, insertions, or substitutions, or modified by other epigenetic changes such as DNA methylations or demethylations.

In addition, large DNA molecules which are linear, circular, or of other topological configurations, obtained either naturally or made in vitro using recombinant methods can be converted into artificial chromosomes by association with certain types of proteins which allow the DNA to be packaged into a compact state. Proteins that have such properties include polyamines such as spermidine, or histone proteins among others. Artificial chromosomes also may be constructed by combining naturally occurring chromosome or chromosome fragments with protein associated DNA made in vitro.

The insertion of artificial chromosomes may be particularly advantageous when the simultaneous introduction of unlinked traits into a recipient cell or organism is desired. In addition, modification of specific nucleotide residues in the naturally occurring chromosomes or chromosome fragments, or in the DNA molecule making up an artificial chromosome, may allow the modulation of the level of expression, or the phenotype expressed by the exogenous genetic information being inserted.

The method described in the present invention for retrieving selected chromosomal material as the source of DNA for transformation is an important and novel aspect of the present invention. It is this aspect of the invention which directly or indirectly brings about differences which distinguishes the present DNA transformation scheme from all other previously described DNA transformation methods, including that found in U.S. Patent No. 4,873,191.

First, the use of chromosome or chromosome fragments to tranfer genetic information makes it possible to introduce very large contiguous segments of DNA. The protein moieties tightly associated with the DNA in chromosomes protect the exogenous DNA from degradation by endogenous nucleases, and also, allows large DNA molecules to remain highly compact, and hence protected from breakage which can result from mechanical shear during the insertion process. Thus with the chromosome transfer method described in the present invention, transomic cells and organisms can be generated which contain the integration of very large DNA fragments of at least 10 megabases or greater. In comparison, the DNA fragments integrated by the injection of naked DNA as descried in U.S. Patent No. 4,873,191 usually is no greater than 50 to 100 kbs in size.

Second, the use of chromosome or chromosome fragments to transfer genetic information provides a much higher efficiency of transformation as compared with the injection of naked DNA. Thus, the inventors have observed the successful incorporation of human DNA with the insertion of just a single copy of a human chromosome fragment into a mouse zygote. While the injection of naked DNA into a fertilized mouse oocyte as described in U.S. Patent No. 4,873,191 usually requires the introduction of many hundreds of copies of the foreign DNA.

Without wishing to be bound by theory, this higher transformation efficiency of exogenous chromosomes and chromosome fragments is likely due to the proteins associated with the chromosomal DNA which afford protection from nucleolytic degradation. In contrast, naked DNA is readily accessible to nucleolytic attack, and is susceptible to rapid degration in many instances before there is an opportunity for incorporation into a recipient cell's genome.

Third, the use of chromosome or chromosome fragments makes it possible to insert the exogenous genetic material not just into the pronucleus of a fertilized oocyte as described in U.S. Patent No. 4,873,191, but in the nucleus of any recipient cell, and in the cytoplasm of any mitotic or meiotic cell. Although naked DNA introduced into the cytoplasm of a dividing cell will likely be degraded by endogenous nucleases, exogenous chromosome or chromosome fragments introduced into a dividing cell, being similar to the endogenous chromosomes present in the mitotic cell cytoplasm, should suffer little or no deleterious effects.

A fourth significant advantage with the transfer of chromosomal material rather than naked DNA is the ability to generate transgenic cells and organisms containing exogenously introduced genetic traits that are not yet biochemically characterized or molecularly cloned. This is especially important in cases where such genetic traits are encoded by genes or a complex of genes which is greater than 200 kbs, as such genetic traits would not be readily clonable with currently available recombinant DNA methods. Moreover, the injection of such large pieces of naked DNA also would not be possible without breakage due to mechanical shear and nucleolytic degradation. Thus the present invention for making trangenic cells and organisms differs significantly from previous U.S. Patent No. 4,873,191 in that it is only in the present invention that methods are disclosed for the production of transgenic cells and organisms expressing traits for which no recombinant DNA clones exist.

The present invention also significantly differs from the chromosome injection study described by Griesbach in several important aspects. First, in contrast to the methods of the present invention where selected chromosomes are retrieved and then inserted into a recipient cell, in Griesbach's study, the chromosomes that are injected represent a random collection of all the chromosomes isolated from the donor cells. Without a selection step to identify the specific chromosomes to be inserted in the recipient cell, a large number of transformants must be generated and then screened to identify the ones that might contain the particular chromosome or chromosomes of interest.

This inefficiency becomes completely unworkable in cases where multiple chromosome or chromosome fragments need to be inserted into the recipient cell. Thus with random chromosome injections, the probability is very low that any recipient cell will have both received the appropriate combination of chromosome(s) or chromosome fragment(s), and incorporated such chromosome(s) or chromosome fragment(s) into its genome.

A second important advantage of the present invention compared to the study of Griesbach is the methods described herein for the retrieval step which allows specific chromosome fragments in addition to selected whole chromosomes to be retrieved for insertion. With random integration, it would be difficult if not impossible to obtain the integration of a precise fragment of any given chromosome. This coupled with the selection step in the present invention removes the random nature of chromosome incorporation as occurs with bulk chromosome injections and allows the selective insertion of targeted genetic material.

Hence with the present invention, not only is the efficiency greatly increased for generating transformants containing specific chromosome insertions, but the present method may be indispensable when it is necessary to insert only a subset of a collection of genetic traits that are colocalized on the same chromosome or when it is necessary to insert genetic traits localized on several different chromosomes.

The collection of novel characteristics of the chromosome transformation method of the present invention lends itself to a number of applications which are not possible with other methods of inserting exogenous DNA for making transgenic cells and organisms.

For example, as the present methods make possible the transfer of pieces of DNA 10 to 30 megabases long, the methods of the invention are ideal for transfer of genetic traits encoded by very large genes, such as the dystrophin gene of molecular dystrophy, and those traits encoded by a complex of genes, such as the human immunoglobulin complex.

For instance, it is well known that for each type of immunoglobulin chain, including $x$ light chains, λ light chains and heavy chains, there exists a separate pool of DNA segments from which a gene encoding a single polypeptide chain is eventually synthesized. Each pool of DNA sequences contains a different set of V region segments located hundreds of thousands of base pairs upstream from one or more C region sequences on a chromosome. In fact, the DNA sequence pools are somewhat more complicated because each V region of the polypeptide chain is encoded by two or three distinct V segments as well as one or more J segments for the light chains and one or more D segment for the heavy chains.

The large DNA segments encoding such polypeptide chains cannot be transferred by another method of DNA transfer. On the other hand, the methods of the present invention are particularly useful for transferring the large DNA segment necessary to encode a functional immunoglobulin or portion thereof. Thus, using the methods of one embodiment of the present invention it is possible to transfer a chromosome or chromosome fragment containing the entire repetoire of immunoglobulin genes from one organism or cell into another organism or cell.

For example, using this embodiment of the methods of the invention, a chromosome or chromosome fragment containing the entire repertoire of V, J and C segments is transferred from a human cell to a recipient cell of another mammal, e.g., a mouse, to generate a transgenic organism having lymphocytes expressing a human immunoglobulin or portion thereof. Such animals would provide access to the entire repertoire of human immunoglobulins and can be used, for example, as a source of B cell lymphocytes to generate human monoclonal antibodies.

More particularly, according to this embodiment of the present invention, chromosomes or chromosome fragments of human chromosomes 14, 2 and 22 which respectively encode heavy chains, kappa light chains and lambda light chains of human immunoglobulins containing the entire repertoire of human V, J, D and C segments can be transferred from a human cell to a recipient cell of another mammal to generate a transgenic cell or organism having lymphocytes which can express a human immunoglobulin or portion thereof.

In this vein, the use of a rodent, such as a mouse, as the recipient organism is a particularly advantageous facet of the present methods for a number of reasons. Firstly, because the human immunoglobulin repertoire is expressed within a "non-human" genetic background, the transomic organism can express "human" antibodies against antigens which are genetically non-polymorphic in man. Moreover allelic exclusion of immunoglobulin genes will ensure the preponderance of B cells make antibodies of only one immune specificity. Secondly, the transomic mice can easily be immunized with a variety of potentially toxic immunogens or antigens and can produce "human" antibodies without the ethical difficulties involved with immunizing human subjects. Thirdly, use of rodents, particularly mice, as the transomic host organisms permits the use of well established cell fusion systems for the production of human monoclonal antibodies from B cell lymphocytes of the transomic mice. Techniques for forming hybridoma cell lines as well as mouse myeloma cell lines are widely known and readily available. See, for example, Coding, 1986, in Monoclonal Antibodies: Principles and Practice, 2d ed., Academic Press, Harcourt Brace Jovanovich, pp. 33-42; 59-77. Additionally, because rodent myeloma cell can be used, any adventitious agents such as viruses which might be present would be of non-human origin. This is an important advantage over systems using EB virus transformed human cell lines because such systems are often contaminated with adventitious agents of human origin, including in particular human oncogenic viral agents. Finally, the ease and the convenience, of using mice and the rapid breeding of mice, i.e., gestation time of about 21 days and first mating at 4 to 8 weeks are additional advantages available using the present methods.

Thus, using the methods of the present invention, transomic mice can conveniently be produced to serve as a source of B cell lymphocytes which can express the whole repertoire of the human immunoglobulin complex. These transomic mice thus serve as a means for easily and conveniently producing large quantities of homogenous human monoclonal antibodies against any desired antigen. Hence, the present methods provide a means for convenient production of human monoclonal antibodies which were not available prior to the present invention.

In practice, the method of one mode of this embodiment of the invention encompasses the following steps:

(a) immunizing with an antigen a transomic animal whose cells (germ cells and somatic cells) contain a human chromosome or chromosome fragment which comprises the genetic material encoding a human immunoglobulin complex or a portion thereof, introduced into a embryonic stage of the animal or an ancestor of the animal by a method comprising : (i) isolating the chromosome or chromosome fragment from a human cell; and (ii) inserting a substantial portion of the chromosome or chromosome fragment of the human cell into the heritable genetic material of the animal or ancestor of the animal by injecting the chromosome or chromosome fragment into a cell of the animal or ancestor thereof;

(b) harvesting B lymphocytes capable of producing antibody against the antigen from the lymph nodes, spleen or peripheral blood of the immunized transomic animal; and

(c) fusing the harvested B cell lymphocytes with a myeloma cell line to produce a hybridoma cell line which produces antibodies against the antigen.

According to a preferred embodiment, the transomic animal is a rodent, particularly a mouse, and the myeloma cell line is a rodent cell line, particularly a murine myeloma cell line. The hybridoma cell line can be propagated in in vitro cell cultures or in vivo as an ascites tumor or a solid subcutaneous tumor in a compatible host animal.

As used in this specification, an antigen is intended to encompass any moiety which is capable of inducing the production of an antibody response, including, but not limited to bacterial, fungal, viral, mycoplasmal, parasitic, histocompatability, tumor and differentiation antigens, etc.

Using the methods of another embodiment of the present invention transomic cells and organisms, including microbes, plants and animals, which produce products of commercial and/or medical value can be generated. For example, organisms and cells can be produced which can serve as efficient, economic sources of products including, but not limited to, hormones, growth factors, clotting factors, enzymes, antibodies etc.

The transomic plants and animals of the present invention also include plants and animals of agricultural value. For example, the transomic animals of agricultural value provided by the present invention include chickens, beef cattle, buffalo, dairy animals, pork, turkeys, lamb, etc. The transgenic plants of agricultural value provided by the present invention include corn, wheat, beets, soya beans, fruit trees, carrots, spinach, asparagus, potatoes, etc.

Thus, using the methods of another alternate embodiment of the present invention, organisms and cells including microbes, plants and animals which possess desirable traits can be generated. For example, plants and plant cells can be generated which possess desirable traits, including but not limited to enhanced drought resistance, pest resistance, herbicide resistance, tolerance of increased or decreased acidity or alkalinity, enhanced growth rate and/or productivity, increased or decreased stature, etc.

As additional examples, animals can be produced having desirable traits including, but not limited to, enhanced disease and/or pest resistance, faster growth and/or reproductive rates, superior meat quality, greater yield of meat or milk, etc. As yet another example, transomic microbial organisms can be produced which can degrade waste and/or toxic materials which can be advantageously employed in environmental control and waste management systems.

Using another embodiment of the present methods, animals and cells can be generated to serve as modeling systems for human and animal diseases and other pathological conditions which are useful for developing drug therapies, diagnostic protocols and reagents and other patient management schemes. Such diseases or pathological conditions can be the result of traits encoded genetically, or they may arise due to effects of infectious agents or environmental agents, or in some cases, the etiology may be unknown.

When the diseases, or pathological conditions are genetically encoded dominant traits with known chromosome locations, then the methods of the invention can be used to generate animal models of the diseases and pathological conditions. The following are offered as illustrative examples of this embodiment and are in no way intended as limiting the scope of this embodiment of the invention.

As an example, Alzheimer's disease is a degenerative neurological disorder which is associated with dementia in 2.5 million individuals in the United States of America. The probability of developing the illness increases to about 50% by the age of 90 in affected families. Genetic evidence suggests that the risk of developing the familial early onset form of Alzheimer's disease is related to the dominant inheritance of a single copy of an affected gene(s) on chromosome 21. Given the rapid aging of the population as a whole in developed countries such as the United States, Canada, and the nations of Western Europe, developing an effective treatment to arrest or cure this degenerative disease is imperative. In addition, there is a great need for better methods and reagents to assist in the accurate diagnosis of Alzheimer's disease. The absence of an appropriate animal model system has, however, hampered such efforts.

Using the method of the present invention, animals such as mice containing an appropriate fragment of chromosome 21 from an individual affected with Alzheimer's disease can be generated. Such animals should serve as useful models for Alzheimer's disease. They will be invaluable for testing pharmacological agents that might be effective in treating this devastating disease. Moreover, the availability of such animal models may make it possible to understand the phsyiological and biochemical basis for this illness, and thus allow the design of more rational approaches for developing effective treatments and diagnostic reagents and protocols.

As another example, Down's syndrome usually results from the inheritance of three copies of chromosome 21 (trisomy 21). This genetic disorder is manifested by a number of problems, including mental retardation, increased risk of leukemia, immunological disorders, premature aging, and the early onset of pathological changes associated with Alzheimer's disease. Down's syndrome is the most common cause of mental retardation. It is found in approximately 0.1% of live births and 0.5% of all conceptuses.

As the incidence for this genetic disorder increases dramatically with maternal age, these numbers are likely to increase given the current trend of two career families in delaying reproduction in the U.S., much of the Western hemisphere and much of Western Europe. Currently there is no treatment for this disease, and its etiology remains unknown. In addition, no good animal model exists for developing effective treatments for alleviating this debilitating illness.

Genetic evidence suggests that the inheritance of a specific segment of human chromosome 21 is responsible for Down's syndrome. Hence, using the chromosome transfer technology of the present invention, it will be possible to generate transomic mice that can serve as models of this illness. The availability of such animals will allow the development of drugs and therapies which could arrest or alleviate the pathological changes associated with this genetic disorder. In addition, the availability of these animals in conjunction with those for Alzheimer's disease will permit the clarification of those common defects which lead to dementia in both illnesses. This information will assist in developing strategies for identifying treatments which might have more efficacy in treating one versus the other disease.

As another example, Huntington's disease is an incurable degenerative neurological disorder associated with late onset, resulting in the loss of productive members of society in the prime of life. This disease is associated with a defective gene(s) on chromosome 4 which is expressed in a dominant manner. Hence, using the chromosome transfer methods of the present invention, to introduce the appropriate fragment from chromosome 4 of an affected individual, it should be possible to generate transomic mice carrying the Huntington's defect. Such animals might serve as models for studying the basis for this degenerative disease, and for developing effective treatments for this lethal illness.

As yet another example, adult polycystic kidney disease is an autosomal dominant genetic disorder associated with the development of cysts in a number of vital organs, including the liver, kidney, pancreas and spleen. This illness is usually not diagnosed until the third or fourth decade of life, and is associated with progressive degeneration of renal function. Without kidney dialysis or transplant therapy, this illness leads to death in the early fifties. Adult polycystic kidney disease is the most common dominant genetic disorder, affecting approximately 1 in 1,000 individuals. This disease is the third most common cause of renal failure in the United States of America and Europe. Thus 9% of all kidney dialysis patients are affected with this disorder.

Despite the prevalence of this disease, currently there is little data on the basis for this degenerative illness, and no known treatment which can arrest or cure the progressive loss of kidney function. There is also no animal model for this disease. However, genetic evidence gathered recently clearly demonstrates that this illness is the direct result of inheritance of a dominant defective gene(s) on chromosome 16.

Hence using the present chromosome transfer technique, the appropriate chromosome fragment from an affected individual can be introduced to an animal such as a mouse, thereby creating an animal model for adult polycystic kidney disease. The availability of an animal model system will be invaluable for developing effective drugs and therapies for arresting or curing this very prevalent illness.

Malignant melanoma is the most rapidly increasing cancer in the human population. Approximately 8 to 12% of such victims are predisposed to this type of cancer due to the inheritance of a dominant trait found on chromosome 1. Currently, nothing is known about the molecular or biochemical basis of this disorder. The gene(s) which leads to increase risk of developing this cancer has yet to be identified. However, using the present chromosome transfer technology, this mutant trait can be introduced into an animal such as a mouse, and thereby generate an animal which could serve as a useful model for studying this disease. Such an animal model would be useful for screening and developing drugs, and in defining other useful patient treatment and management schemes.

As other illustrative examples, genetic abnormalities such as those associated with single mutant genes or chromosome abnormalities and the metabolic diseases that have been mapped to specific autosomes such as those mentioned, respectively in Tables 37-1 and 34-3 in James B. Wyngaarden and Lloyd H. Smith, eds., Cecil Textbook of Medicine Vol. 1, 18th ed., W.B. Saunders Company, p. 169, 156 (1988), the disclosure of which is incorporated herein by reference.

Using another embodiment of the present methods, animals and cells also can be generated that will assist in developing drug therapies, diagnostic protocols and/or reagents, and other patient management schemes for pathological conditions arising from autoimmune diseases, cardiovascular diseases, infectious diseases such as AIDS or hepatitis, or other pathological conditions of known and unknown origins. Thus using the methods of the present invention, transomic cells or animals can be made which contain chromosome(s) or chromosome fragment(s) which will impart to the cell or animal characteristics that are useful for modeling the various pathological conditions. One example is the introduction of human genetic material into cells or animals which would allow the HIV virus associated with AIDS to infect and reproduce in a transomic nonhuman cell or animal. Such cells or animals could serve as an invaluable model system for developing drugs or reagents to treat or diagnose AIDS infections.

Using still another embodiment of the present invention, transomic organisms and cells, including microbes, plants and animals can be generated which can serve as a human chromosome library. For example, by generating a collection of transomic mice or mice cells, each containing a different segment of human chromosomal material, it should be possible to build up a "library" of animals or cell lines which would cumulatively contain all of the chromosomes of the human genome.

Such a collection of animals or cell lines would not only be invaluable for research purposes, but also have very practical applications for the biotechnology/pharmaceutical industry. It would greatly speed up current attempts at mapping the human genome, as it would readily provide genetic linkage data which currently could only be obtained by much more laborious methods. Perhaps of immediate interest would be the potential of using such a library to obtain linkage data for expediting restriction fragment length polymorphisms (RFLP) analysis for mapping the chromosomal location of genes linked to specific human diseases. In addition, it is possible that some of these animals or cell lines might also serve as models of other human diseases or pathological states, including those which may arise due to gene dosage effects.

Aside from making human chromosome libraries, the methods of the present invention also can be used to generate similar chromosome libraries from any cell or organism, and such chromosome libraries can be made either as a collection of transomic cell lines or transomic organisms. Thus the chromosome library can be of plant, animal or microbial origin, and the recipient transomic cells or organisms also can be derived from or consist of plants, animals or microbes. Aside from other applications, chromosome libraries of plants and animals of agricultural importance, or of other commercial value, can be used to assist in mapping and breeding in of desirable traits, or the mapping and breeding out of undesirable traits.

In another embodiment of the present methods, transomic animals, plants, cells and microbes can be generated in which one or more endogenous chromosome region(s) is replaced by one or more exogenous chromosome fragment(s), through which a disease or pathological state caused by the genetic defect(s) contained within the replaced chromosome region(s) is cured or alleviated. In addition, such chromosome replacement strategies can be used to introduce desirable traits in place of endogenous chromosome regions encoding traits which may be less desirable.

For haploid organisms, the replacement of endogenous genetic material in the recipient organisms would result in curing or alleviating of the genetic defect(s), which may include the abatement of certain disease or pathological states, or result in substitution with the desirable traits. For diploid organisms, if the defective or undesirable trait(s) are recessive, then affected individiuals are homozygous for the defective or undesirable trait(s), and the replacement of the chromosome segment(s) encoding the defective or undesirable trait(s) in just one of the two chromosome homologs will alleviate, cure, or otherwise appropriately modify the organisms. For diploid organisms in which the defective or undesirable trait(s) are dominant and are found in the homozygous state, then either double replacements must occur to cure the affected organisms, or breeding, mating, or somatic recombination after a single replacement event can give rise to cured progeny. For diploid organisms in which the defective or undesirable traits are dominant and the affected individual is heterozygous, replacement of just the one chromosome containing the defective or undesirable trait will cure, alleviate or appropriately modify, the genetic defect or undesirable trait. This same scheme can be extended to triploid and higher ploidy organisms, which is especially relevant for example, with various plant species which can exist in different states of chromosome ploidy.

The replacement of an endogenous chromosome segment in the host organism with an exogenous chromosome fragment may occur if the insertion of the exogeneous chromosome fragment is mediated by some sequence homologies between the endogenous and exogenous chromosomes. This homology facilitated recombination process would be more likely to occur with the introduction of large segments of genetic material as mediated by chromosome fragment injection, as compared to the relatively small amount of genetic material introduced by DNA injection (megabase quantities as compared to kilobase quantities; at least 1,000 fold difference in amount).

It has been shown previously that the amount of sequence homology directly affects the rate of homologous recombination for targeting exogenous sequences into a host genome, with 20 fold increase in homologous recombination detected with 2 fold increase in sequence homology (see Capecchi, Trends in Genetics (1989) 5:70-76). Therefore, the replacement of the endogenous chromosome regions by the exogenous chromosome fragment is more likely if a chromosome containing extensive regions of homology is introduced, with the degree of homology present in the exogenous fragment determining the efficiency of endogenous chromosome replacement(s) rather than exogenous chromosome insertion in an endogenous nonhomologous chromosomal site. Moreover, the inventors' own data with the injection of human centrometric fragments containing human satellite DNA sequences into mouse embryos suggests that homologous recombination events may occur readily in the recipient cell. Thus the inventors have observed the incorporation of exogenous human centromeric chromosome fragments into the mouse centromere which contains a related mouse satellite DNA sequence.

As used herein the term chromosome comprises the genetic material of an organism. In accordance with this invention, chromosomes include the genetic material of prokaryotic and eukaryotic organisms whether or not comprised of DNA, including the RNA genetic material of, for example, RNA viruses. In accordance with this invention, as discussed supra, chromosomes include at least some of the proteinaceous, carbohydrate and other materials generally associated with the foregoing genetic materials.

It will be understood by persons of ordinary skill in the art that the foregoing nucleic acid and other compositions may be included in chromosomes to greater or lesser degrees depending upon the source and identity of the chromosomal materials. Chromosome fragment means part of a chromosome, that is less than the entire chromosome. A chromosome fragment may contain part of a gene, one gene, or more than one gene, or any combination of genes and parts of genes. Heritable genetic material means genetic material of the organism that is passed to later generations of the organism through sexual or asexual

EP 0 375 406 A2

reproduction.

In the context of this invention, chromosomes do not include submicroscopic plasmids and recombinant nucleic acids contained in such plasmids. Notwithstanding this definition, however, artificial chromosomes constructed by combining nucleic acids with proteins, including small submicroscopic plasmids and recombinant nucleic acids combined with proteins, are comprehended within the definition of chromosome for the purposes of this invention.

In addition, organisms whose genetic material contain nucleic acids propagated from artificial genetic materials such as plasmids and other recombinant nucleic acids may provide chromosomes or chromosome fragments as comprehended within the definition of chromosome for the purposes of this invention.

As used herein, the term organism includes one-celled organisms such as bacteria and protozoans; viruses; fungi; and multicellular organisms, such as plants and animals, as well as single cells, tissues and tissue cultures of multicellular organisms.

Chromosomes used in the methods of the invention may be derived from any prokaryotic and eukaryotic organisms. Chromosomes from mammalian, avian, plant and microbial organisms are examples of suitable sources of genetic material for use in the invention. Recombinant DNA or recombinant DNA inserted within chromosomes is also a suitable source of genetic material.

The particular chromosome or chromosome fragment to be transferred will depend on the genetic information that is to be transferred. The chromosome or chromosome fragment of interest can be identified and selected by methods known in the art for locating genes or DNA in chromosomes.

Thus chromosomes may be identified by distinctive shape or size, karyotype analysis, in situ hybridization analysis, and other standard methods for chromosome staining and banding. Alternatively, the chromosome of interest can be purified by biochemical methods for fractionating chromosomes, such as eletrophoretic, centrifugal and light activated sorter methods. For artificial chromosomes, the chromosome can be constructed with DNA molecules previously identified as coding for the gene or genes of interest.

The chromosome and chromosome fragments useful in the practice of the invention generally are (substantially) larger than 100 kilobases (kbs), preferably greater than 250 kilobases; more preferably greater than 500 kilobases; most preferably greater than 1 megabases. Chromosome fragments of 0.3 to 1.0 μm, corresponding to 10 to 30 megabases, are easily transferred using the methods of the invention. It is to be understood, however, that chromosomes or chromosome fragments less than 100 kilobases or greater than 30 megabases are also suitable for use in the methods of the invention.

The recipient organism may include many types of eukaryotic and prokaryotic organisms. In the embodiments of the invention whereby transomic cells are produced, examples of suitable types of cells are mammalian, avian, plant and microbial cells. Tissue cultured cells from plant, mammalian or other sources are also suitable for use.

In the embodiments of the invention whereby transomic animals are produced, one-celled fertilized or unfertilized oocytes, preferably non-human oocytes, sperm and germ cells, also preferably non-human, and other embryonic cells of the embryo, including pluripotential embryonic stem (ES) cells (vide infra), are suitable for use in the invention. In preferred embodiments of the invention, the recipient cells are non-human embryonic cells; more preferably non-human mammalian embryonic cells such as mouse embryonic cells.

Before injection of the chromosome or chromosome fragment into the recipient cell, the chromosome or chromosomes of the donor organism are prepared so that the chromosome or chromosome fragment of interest can be selected and retrieved. The chromosome or chromosomes are preferably prepared by spreading the chromosome or chromosomes of the donor organism on a surface to which chromosomes will not adhere irreversibly (referred to herein as "nonadherent") to any significant degree, such as treated glass or a plastic material. The surface itself may be in any shape, such as slides or tubes or other shape of glass or plastic. A suitable non-adherent surface is siliconized glass.

One suitable method for siliconizing a glass surface for use in the methods of the invention is dipping glass slides into a solution of Prosil-28 (Thomas Scientific) diluted 1:100 in water. The slides are then rinsed in water and left to air dry. Other methods of treating a glass surface which makes it nonadherent are also suitable.

The chromosomes may be spread on the surface by conventional methods. A suitable method entails spreading hypotonically shocked cells, which are the source of chromosomes, onto siliconized glass.

Prior to spreading onto a surface, the hypotonically shocked cells also may be mixed with a fixative, then spread onto siliconized glass in the fixative, with the fixative subsequently being removed by evaporation. A variety of fixatives may be used in this procedure, some example include methanol, ethanol, mixtures of methanol:acetic acid or ethanol:acetic acid in a range of proportions (preferably from 20:1 v/v to

15

2:1 v/v). A preferred fixative is a solution comprised of 2:1 v/v mixture of methanol:acetic acid. As a result of the spreading procedure, the chromosomes are immobilized on the surface suitable for the subsequent selection and/or retrieval steps.

If eukaryotic cells are used as the source of chromosomes, they are preferably in the mitotic stage at the time of chromosome isolation. During this stage of the cell cycle, chromosome identification and manipulation are easier than at other stages in the cell cycle as mitotic chromosomes are usually organized in a more compact state. To enrich for mitotic cells, donor cells can be blocked in mitosis by conventional methods such as treatment with colcemid or colchicine. Alternatively, mitotic chromosomes can be obtained from cells that are synchronized in the mitotic stage of the cell cycle. Cell cycle synchronization can be achieved by using a variety of standard methods, such as with thymidine block, hydroxyurea treatment, or by cell elutriation or light activated cell sorting methods to purify mitotic cells from cells in other stages of the cell cycle.

In some embodiments of the invention, prior to spreading to the chromosomes on a surface, the chromosomes may be first harvested as a bulk mixture which is then further fractionated by electrophoretic, centrifugal, or light activated sorter mediated methods, or other methods which can enrich or purify a selected chromosome(s) which is to be retrieved for insertion. The appropriate purified fraction of chromosome(s) can then be spread on a surface for further isolation and retrieval using the method of the present invention.

After the chromosomes are spread onto a surface, when necessary, they may be visualized to facilitate selection and/or retrieval of the desired chromosome or chromosome fragment. The visualizing step of the methods of the invention can comprise staining the chromosomes with a stain substantially non-toxic to the recipient cell. As used herein, the term non-toxic stain refers to a stain which does not degrade DNA or significantly decrease the viability of the recipient cell. Examples of such stains include basic Fuchsin, Griemsa or Wright's stain prepared by conventional methods. As an alternative to staining, the chromosome or chromosomes of the donor organism may be visualized by standard light microscopic techniques such as Hoffman modulation, differential interference contrast optics, Nomarsky, and phase contrast optics.

For the selecting step, in some embodiments of the invention, prior to spreading, chromosomes may be fractionated by electrophoretic, centrifugal, light activated sorter mediated methods, or other fractionation schemes to enrich or purify the chromosome(s) which is to be retrieved for insertion. The appropriate purified fraction of chromosome(s) can then be spread on a surface, such as a slide or a test tube, for further isolation and retrieval using the methods of the invention.

For the selecting step, any of a number of different chromosome identification methods can be used, including standard chromosome staining methods, such as banding with Giemsa or Wright's stain. Given that the banded karyotype of each species of organism is unique, the characteristic chromosome banding pattern can allow accurate chromosome identification in the selecting step. However, optical methods for visualizing unstained chromosomes also can be used for chromosome identification, as some chromosomes in some organisms have unique shapes associated with characteristic ratios of arm lengths. Some suitable optical methods for visualizing unstained chromosomes can include differential interference contrast optics, Nomarsky optics, Hoffman modulation and phase contrast optics among others.

Another suitable method for chromosome identification in the selecting step is the use of chromosome in situ hybridization with nucleic acid probes specific for sequences known to be present in the chromosome or chromosome region of interest. Alternatively, staining of chromosome with antibody reagents specific for antigens known to be present only in the chromosome or chromosome region of interest also can be used for the selecting step. Such antigens can include nucleic acids, proteins, carbohydrates or other chemical moieties.

After the selecting step, a chromosome or chromosome fragment is retrieved. A fragment of chromosome can be retrieved, preferably by cutting a chromosome containing the desired chromosome region with a glass pipette and displacing the chromosome fragment relative to the original chromosome from which it was cut. Other methods of cutting nucleic acids, such as using a needle, laser or enzyme, are also suitable for use in the invention.

In embodiments of the invention wherein an entire chromosome is transferred into the recipient cell, cutting a fragment is not necessary and retrieval will comprise positioning the chromosome so that it can be manipulated into the pipette. This might comprise, for instance, moving the chromosome away from the other chromosomes on the surface. In embodiments of the invention in which the method of preparation of the chromosomes of the donor organism results in fragmented chromosomes, the fragment of interest may also be retrieved without cutting.

The fragment of chromosome cut from the chromosomes, or an entire chromosome, is then manipulated into a pipette. This is preferably accomplished by contacting the chromosome or chromosome

16

fragment with a mobilizing solution and aspirating the chromosome or chromosome fragment into a pipette. Thus the mobilizing solution is used to facilitate aspiration of the fragment into the injection pipette, or another suitable device as described supra.

In preferred embodiments of the invention in which the spreading step results in the chromosome or chromosome fragment being immobilized on a surface, the mobilizing solution of the invention preferably comprises protease and may also contain other mobilizing agents such as ethylenediaminetetraacetic acid (EDTA), polyvinylpyrrolidone, and non-toxic stain. A preferred protease is trypsin. Other proteases may also be suitable for use in the mobilizing solution. A preferred non-toxic stain is basic Fuchsin. Other suitable stains would include Giemsa or Wright's stain. The concentration of the constituents of the mobilizing solution may vary, generally ranging from about 0.01 wt.% to about 0.06 wt.% trypsin and from about 0.01 mM to about 1.0 mM EDTA; preferably 0.025 wt.% trypsin and 0.1 mM EDTA.

The mobilized chromosome or chromosome fragment, with the mobilizing solution surrounding it, is then aspirated into an injection pipette or another suitable device as described supra. The mobilizing solution is then diluted by aspirating into the tip of the injection pipette a physiologically acceptable liquid, while at the same time keeping the chromosome or chromosome fragment in the pipette. This manoeuver may be done repeatedly to sufficiently dilute the mobilizing solution with the physiologically acceptable liquid.

In some other embodiments of the present invention, the mobilized chromosome(s) or chromosome fragment(s), instead of being loaded into an injection pipette, can be loaded into a microelectrode or encapsulated in lipid vesicles (see, e.g., Paphadjopoulous et al, In Vitro (1980) 16:49-54 which is hereby incorporated herein by reference) in the presence of a physiologically acceptable liquid. Alternatively, the chromosome(s) or chromosome fragment(s) can be loaded onto microprojectiles, as discussed supra.

The physiologically acceptable liquid may be any liquid which is not significantly harmful to the recipient cell. In the event that protease is included in the mobilizing solution, the physiologically acceptable liquid preferably contains at least one protease inhibitor. Preferred protease inhibitors are epsilon-amino caproic acid and benzamidine hydrochloride. Examples of suitable liquids are salt solutions and protein solutions.

For insertion of a chromosome(s) or chromosome fragment(s) into mammalian cells, a preferred physiologically acceptable liquid is a potassium chloride solution, more preferably a solution of about 50 millimolar potassium chloride. A more preferred physiologically acceptable liquid is a solution of about 10 mM Tris, about 0.1 mM EDTA and about 0.1% polyvinylpyrrolidone containing about 50 mM epsilon amino-caproic acid and about 5 mM benzamidine hydrochloride. A protein solution containing bovine serum albumin is also suitable for use in the methods of the invention and may be preferred in some cases. The specific physiologically acceptable solution to be used will vary and will be adjusted to suit to the recipient cell type.

In some embodiments of the invention the mobilizing solution may be a physiologically acceptable liquid. In these embodiments, the step of diluting the mobilizing solution with a physiologically acceptable liquid will not be necessary, and this step can be eliminated.

When the recipient cells are embryonic animal cells, it is preferable that the embryonic cells be one-celled fertilized oocytes. Fertilized oocytes are eggs which have been activated to initiate embryonic development. The activation can be achieved by sperm fusion with the egg, but other methods of activation are also possible such as egg membrane perturbation by mechanical pricking, or electrical stimulation.

Introduction of the exogenous genetic material at the fertilized oocyte stage will greatly increase the likelihood that the genetic material will be incorporated into the germ cells and somatic cells of the transomic animal. Such injected oocytes also can be used to generate embryonic stem cell lines from which animals can be obtained that will provide germ line transmission of transomic strains containing the exogenous chromosome fragment.

The presence of the exogenous genetic material in the germ cells of the transomic founder animal in turn means that at least some, or all, of the founder animal's descendants will carry the exogenous genetic material in all of their germ cells and somatic cells. Introduction of the exogenous genetic material at a later embryonic stage might result more frequently in the exogenous genetic material's absence from some somatic or germ cells of the founder animal, but descendants of such an animal that inherit the exogenous chromosome or chromosomes will carry the foreign genetic material in all of their germ cells and somatic cells.

The methods of the invention are practiced with conventional apparatus for microinjection, such as those described in Gordon, J.W. and Ruddle, F.H., "Gene Transfer into House Embryos: Production of Transgenic Mice by Pronuclear Injection", Methods in Enzymology, Vol. 101, pp. 15 4411-433, Academic Press, 1983 and Hogan et al., Manipulating the Mouse Embryo, A

Laboratory Manual, Cold Spring Harbor Laboratory, Cold Spring Harbor, New York, 1986. Pipettes used in the methods of the invention are prepared according to methods known in the art, such as the methods in Gordon and Ruddle supra or Hogan et al. supra., with modifications as described herein.

Generally, injection of the recipient cell with the chromosome or chromosome fragment is performed under 400x magnification. Injection of cells not attached to a substratum will require first immobilizing the recipient cells, such as by suction using a holding pipette. The recipient cell is then pierced with an injection pipette containing the chromosome or chromosome fragment and the chromosome or chromosome fragment is ejected into the cell, either into the nucleus of the cell, or the pronucleus of the fertilized oocyte, or into the cytoplasm of a dividing mitotic or meiotic cell or oocyte.

Once the recipient cells have been injected with the chromosome or chromosome fragment, the cells are cultured using conventional techniques in an appropriate medium for the type of cell. In the case of mouse embryos (i.e., fertilized oocytes and later development stages), the recipient cells can be either directly transferred to the oviduct or uterus of a pseudopregnant female mouse (mother), or preferably of a pregnant female mouse (mother), or they can be first cultured for one or more days and then implanted either in the oviduct or in the uterus of a pseudopregnant female mouse, or preferably of a pregnant female mouse, for growth and development until birth. The oviduct transfer can be carried out for embryos from the 1 cell stage to the 4 to 8 cell stages, while the uterine transfer can be carried out for embryos from the 8 cell stage to the blastocyst stage.

The transomic embryos alternatively can be cultured to isolate transomic pluripotential embryonic stem cell lines (Evans et al, Nature, (1981) 292:154-156; Martin, Proc. Nat. Acad. Sci. (USA) (1981) 78:7634-7638). Such transomic stem cells can be injected into blastocysts stage mouse embryos, followed by transfer in utero to a pseudopregnant female for growth and development until birth. Animals obtained in this manner can provide germ cells derived from the transomic embryonic stem cells, and thus give rise to transomic offspring which are transomic in all of its somatic and germ cells.

Thus in another embodiment, the present invention provides pluripotential embryonic stem cells. Starting from transomic embryos obtained by the present methods, pluripotential embryonic stem cells can be made using standard methods (Evans et al, supra; Martin, supra.

For example, this entails allowing embryos to grow to the blastocyst stage in vitro, and after hatching from the zona pellucida and attaching to the culture dish substratum, isolating the uncovered inner cell mass (ICM) clumps from embryos in which the trophoblasts cells have spread out onto the culture dish.

The isolated inner ICM clumps are then briefly trypsinized, and forced through a small pipette to break up the clumps into smaller cell aggregates, and such aggregates of cells are then plated on a mitomycin C treated γ-irradiated fibroblast feeder layer, such as that comprised of primary mouse embryonic fibroblast cells or mouse fibroblast STO cells. After 2 days, embryonic stem cell colonies are apparent, and they are characterized by having very large nuclear/cytoplasmic ratios, with overall cell size being small, and with cells in colonies not having distinct cell borders.

Such embryonic stem (ES) cell lines can be maintained indefinitely in culture, and upon transfer into the blastocyst of a mouse embryo, will participate in normal development, and upon transfer in utero will provide a chimeric founder mouse which in some cases will contain cells derived from the exogenous embryonic stem cells in all three germ layers, including cells in the germ line. Thus progeny from such mice can provide a means for establishing mouse strains containing the genetic makeup of the ES cell lines.

Hence, by beginning this process of ES cell isolation using chromosome fragment injected mouse eggs, transomic ES cell lines may be generated containing the heritable incorporation of selected exogenous chromosome or chromosome fragments, and chimeric mice generated with blastocysts injected with such ES cells can, upon breeding, provide mice that will contain the exogenous chromosome or chromosome fragment as a result of germ line contribution from the transomic ES cells in the chimeric founder mouse.

The production of ES stem lines as outlined above can be invaluable in reducing the cost and the amount of time otherwise needed to generate animals containing the desired exogenous chromosome insertion. Hence, by first identifying in culture the ES cell lines which contain the chromosome insertion of interest, the appropriate transgenic mouse strain can be created using standard chimeric embryo methods without the need for generating and screening many different transomic mouse strains. The use of ES stem cell lines can be extended to animals other than mice, and among other animals, can be easily applied to all mammalian embryos. It would be particularly useful in introducing genetic traits into large animals such as farm animals, as the production of transgenic farm animals is much more cumbersome and costly than that of making transgenic mice.

Using ES cells, transomic animals of commercial value may be generated in which germline transmission of the exogenous genetic material is not obtained, or in which the somatic and/or germ cells are mosaic such that not every cell contain the exogenous chromosome or chromosome fragment. Similarly, transomic animals made without the use of ES cells also may be mosaic as a result of other reasons, and such animals nevertheless are likely to be suitable for many of the various applications stated previously for transomic animals. Thus aside from mosaicism arising from the use of ES cells for generating a transomic animal, mosaicism also may arise from the making of transomic animals by aggregating blastomeres from one or more mouse embryo containing the insertion of an exogenous chromosome or chromosome fragment with blastomeres from one or more uninjected mouse embryos. Another possible basis for mosaicism is if the exogenous chromosome or chromosome fragment is not integrated at the time of injection, but after one or more rounds of cell division. Yet another possible basis for mosaicism is if the exogenous chromosome or chromosome fragment might be deleted in some cell types after it has become incorporated into the recipient cell genome. These and other reasons can directly or indirectly result in mosaicism in the transomic animals generated. This mosaicism may be observed in the germline and/or somatic tissues, and in the extreme, may result in the absence of any somatic or germ cell containing the exogenous chromosome or chromosome fragment.

Nevertheless, it is important to note that such mosaic transomic animals may be useful for many of the various commercial applications discussed above. For example, even if not every B cell lymphocyte in a mouse contains the exogenous chromosome fragments encoding the human immunoglobulin genes, such a mouse might nevertheless be useful for the production of human antibodies as described above. Similarly, mosaic transomic animals may be suitable as disease models, etc. In fact, in some cases, a mosaic transomic animal actually may be advantageous, if for example, the exogenous genetic material were to have a deleterious effect on the animal's health and viability if present in every cell of the animal. Note than even if mosaicism results in the absence of germ line transmission, the availability of ES cells containing the insertion of an appropriate exogenous chromosome or chromosome fragment may be particularly useful in facilitating the making of additional transomic animals.

For mouse embryo applications, the methods of Gordon, J.W. and Ruddle, F.H., "Gene Transfer into Mouse Embryos: Production of Transgenic Mice by Pronuclear Injection", Methods in Enzymology, Vol. 101, pp. 411-433, Academic Press, 1983 and Hogan et al., Manipulating the Mouse Embryo, A Laboratory Manual, Cold Spring Harbor Laboratory, Cold Spring Harbor, New York 1986, both of which are specifically incorporated herein by reference, are suitable for use in the invention with modification per the above description.

In the case of embryonic cells from other species, after injection with the chromosome or chromosome fragment, the embryos are transferred into pseudopregnant females, or preferably pregnant females, or cultured and then transplanted into pseudopregnant females, or preferably pregnant females, or otherwise treated according to the requirements of the species, using methods known in the art.

In a preferred embodiment of the invention, recipient cells into which the chromosome or chromosome fragment has been injected are implanted either in the oviduct or in the uterus of a pregnant female for growth and development until birth using standard techniques. As shown in Example 3 supra the inventors discovered that pregnant females provide a notable enhancement in embryo yield as compared to pseudopregnant females. In Example 3 an increase in yield of more than 100% was observed with pregnant females as compared to pseudopregnant females.

Thus a salient advantageous embodiment of this invention for facilitating embryo development to term is the use of pregnant females. These pregnant females are females that are mated with normal males and therefore are pregnant with their own litters, rather than pseudopregnant females which are generated by mating females with vasectomized males as conventionally used in making transgenic animals with DNA injection methods. This difference is important in providing a greater efficiency of live births from the chromosome fragment-injected embryos.

The presence of the endogenous litter in the pregnant female facilitates the pregnancy, probably by providing an appropriate hormonal balance that might make the uterine epithelium more receptive to implantation and support further embryonic development. The pregnant females may be caged with normal males, and used on the afternoon of the day in which a vaginal sperm plug is detected, or anytime within the first five days of pregnancy. The precise time chosen for embryo transfer depends on the development stage of the exogenous embryo.

In most cases, such females will contain embryos derived from fertilized eggs of their own, the number ranging from 0 to 16, depending on the age of the female, and the strain. Using CD1 females of 8 to 12 weeks of age, usually 12 to 16 embryos are found present in such mated females.

To distinguish fetuses or mice derived from the exogenously introduced chromosome fragment injected oocytes or embryos from that derived from the endogenous uninjected embryos, genetic markers can be used, such as differences in coat color and eye pigmentation. For example, the surrogate CD1 (albino) females, when mated with B6D2F1 males (black in coat color), will give rise to fetuses and animals with darkly pigmented eyes, and coat color. If the exogenously introduced embryos are derived from Swr/J (albino) females mated with SjI/J (albino) males, then the resulting fetuses and animals will have red eyes, and white coat color indicative of their albino status. In this manner, albino mice derived from the chromosome fragment injected eggs will be easily distinguishable from the pigmented mice derived from the endogenous embryos. Other genetic markers also can be used, such as isozyme markers, histocompatibility antigens, etc. or other morphologically distinct characteristics such as kinked tail, etc. or behavior modifications, or any other distinguishable characteristics that can be directly or indirectly observed or assayed.

The present invention also may be used to generate cell lines from transomic animals or plants which were made using the methods of the present invention. Such cell lines can be generated using standard methods for cell isolation, and propagated under standard culture conditions. Such cell lines can be used in various applications, including modeling of diseased or pathological states, production of biological compounds, the analysis of genetic linkage, among others.

The methods of the present invention also may be used to generate transomic cell lines by injecting chromosome or chromosome fragments directly into tissue culture cells. This may include animal, or plant tissue culture cells. The use of tissue culture cells as recipients is of special significance for plants, as whole plants can be readily regenerated from tissue culture cells under defined conditions (see infra). Similarily, the direct injection of chromosome or chromosome fragments into pluripotential animal embryonic stem cell can be used to generate transomic stem cells from which transomic animals can be generated.

Plant cells suitable for use as recipient cells in the invention are cells such as meristematic cells, germ cells, callus tissue, microspores, pollen, and somatic or zygotic embryos. Some plant cells have walls that are thick or rigid because of cellulose or other structural molecules. When these cells are recipient cells, they may need to be converted into protoplasts by removal of the structural molecules using conventional methods such as enzyme digestion, prior to inserting the exogenous chromosome or chromosome fragment.

With cells in which the cell wall has not been removed, pipettes (or other suitable device, supra) with greater rigidity can be used to allow cell wall penetration. Also laser mediated and other methods, such as microprojectile mediated puncturing of a region of the cell wall may be used to facilitate chromosomal insertion. Localized enzymatic degradation of the cell wall may also be used to facilitate the insertion or injection process. Adjustments in the apparatus or other conditions used for immobilizing the cell for injection, or other steps of the methods may also be necessary. It is within the scope of the invention to produce transomic plants from the transomic plant cells produced by the methods of the invention.

Transomic organisms of plant origin provided by the present invention may be obtained, for example, by regeneration of the plant from somatic tissues or a callus comprising transomic cells. The regeneration of normal plants from somatic tissues is a well known technique. See, e.g., Horsch et al, Science (1985) 227:1229-1231, this document being hereby incorporated herein by reference. For example, many plants can be readily regenerated from a small disc of somatic tissue punched out of a leaf and placed on a moist nutrient medium. Regeneration of a complete plant is also possible from a callus (a disorganized, undifferentiated cell mass originally excised from a mature plant and then grown indefinitely in tissue culture). Treatment with a proper plant hormone induces differentiation of the callus into roots and shoots, and eventually a complete plant with fertile flowers can be regenerated.

The methods of the present invention are useful to obtain both somatic tissues and calluses containing transomic cells. These are then used to regenerate complete transgenic plants.

A major problem associated with the use of genetically engineered Ti plasmids for crop improvement is that many important crop plants or monocots are thought to be resistant to infection by Agrobacteria. Although some recent evident suggests that Agrobacteria can infect some monocots such as lily and asparagus, important plants such as corn and wheat have not yet been successfully infected with Agrobacteria.

The method of the present invention advantageously does not depend upon the infectability of target cells by Agrobacterium or other vectors, and thus permits producing transgenic plants of important crop plants, and provides a ready method for economically infecting important monocots.

Although the previous study of Griesbach discusses the microinjection of bulk isolated chromosomes for making transgenic plants, there are a number of important differences which distinguishes the methods of the present invention from that of Griesbach (see discussion supra). One of the major points of comparison which differentiates the present invention is our procedure for the selection and retrieval of defined chromosome regions for insertion, as compared with the injection of a random collection of a complete chromosome mixture isolated from Petunia in Griesbach's study.

The ability to specify the genetic material to be transferred has important bearing on the probability and efficiency for obtaining transgenic plants with desired traits, without which the chromosome injection method is not economically viable for commercial applications, nor likely to be useful even for research purposes. However, the study of Griesbach appears to document the feasibility of microinjecting chromosomes into plant protoplasts, and such cells are capable of regenerating into complete plants with full reproductive function. Thus using the methods of the present invention, plants with defined genetic traits can be generated, and will be invaluable for engineering horticultural and agricultural plants with commercially desirable traits.

In another embodiment, the present invention provides transomic plants capable of producing gene products corresponding to genes of microbial, plant or animal sources. It has already been shown that transgenic plants are capable of producing animal antibodies (cf. Hiatt et al, Nature (1989) 342:76-78). Hiatt et al transformed tobacco plants using Agrobacterium containing various plasmids. The transgenic tobacco plants obtained were then found to be capable of producing a specific antibody. The present invention is distinguished from Hiatt et al at least in that the method of the present invention allows the insertion of chromosome or chromosome fragment from the donor organism into the recipient plant organism, thus permitting adding to a recipient plant cell a much larger DNA molecule as noted supra. Thus, the methods of the present invention will allow the transfer of genetic information which encodes the entire repertoire of antibody genes, not just a single antibody gene.

It is also within the scope of the invention to transfer RNA to recipient cells. For instance, this could be accomplished by transferring chromosomes during a time wherein the genes are actively transcribed such that many copies of the corresponding RNA would be complexed with the chromosome or chromosome fragment and would be transferred with it. Alternatively, RNA which might serve as the source of genetic information could be transferred alone.

The following examples are offered by way of illustration and not by way of limitation. The invention has been described herein with reference to certain preferred embodiments and examples, however obvious variations will be apparent to those skilled in the art, the invention is not to be considered limited to the particularly exemplified embodiments.

## EXAMPLES

### Example 1

#### Preparation of Chromosomes:

Condensed chromosomes from a human fibroblast cell line, MRC-5 were used as the source of chromosomes and chromosome fragments. MRC-5 is a diploid fetal lung derived fibroblast cell line that has a normal karyotype. The cells were blocked at metaphase with colcemid (10 ug/ml) for two hours, then processed for making metaphase spreads as follows. The cells were harvested by a brief trypsin/EDTA (0.05%/0.5 mM) treatment (to dislodge the cells from the petri dish), spun down by brief centrifugation in a table top clinical centrifuge, then resuspended in 0.075 M KCl for 15 to 20 minutes at 37° C. After this hypotonic shock, the cells were spun down again and resuspended in ice cold methanol/acetic acid (3:1). After 5 minutes in this fixative on ice, the cells were spun down, then again resuspended in fresh ice cold fixative.

After thirty minutes of further incubation on ice, the cells were resuspended in a very small volume of fresh cold fixative, and spread onto glass slides. This involves dropping small aliquots of this cell suspension onto siliconized glass slides (from a Pasteur pipette), and immediately vaporizing the fixative by heating the slides over a gentle stream of steam from a boiling water bath. For the MRC-5 cell line, it is likely that spreads obtained during the early phases of this spreading procedure are most suitable for dissection and injection. Possible limitations include the fact that cells fixed for greater than forty five minutes to one hour often do not break open on the siliconized glass slides, and those that do break open appear to be unsuitable for dissection/injection (tend to swell during dissection, and difficult to remove from the glass slide as a result of increased adhesiveness to the slide). Also, high humidity levels in the ambient environment greatly reduces the efficiency with which well formed chromosomes are obtained. This can be alleviated to a large extent with the use of a high capacity dehumidifier.

Preparation of Dissection, Injection and Holding Pipets:

Dissection pipettes with 1-2 um tip size were made from Kwikfil glass capillaries (1.0 mm o.d., 0.58 mm id., W.P. Instruments), pulled on a vertical Narishige puller. For injection pipettes, glass capillaries (1.0 mm o.d., 0.8 mm i.d., V.W.R. Scientific) were cleaned with chromic acid, and then baked in a 160°C oven for 10 minutes. Once cooled to room temperature, the capillaries were treated with Nonidet P-40, rinsed thoroughly and air dried. These capillaries were pulled on a horizontal puller (Industrial Science Associates, Inc.) to obtain a tip size of about 0.1 um. Prior to injection, pipette tips were enlarged by gentle tapping on the holding pipette to achieve a final tip size of 0.5-1.0 um. For holding pipettes (80-100 um o.d.), glass capillaries were hand-pulled over a microburner, placed on a Defonbrune-type microforge, broken on a glass anvil, and polished.

Dissection of Chromosome Fragments:

Chromosome fragments were dissected from stained metaphase chromosome spreads (0.1% basic fuchsin) obtained from a normal human fibroblast cell fine, MRC-5. The spreads were stained for one hour with 0.1% basic Fuchsin, rinsed with water and then air dried. Fragments of 0.3-1.0 um in size (corresponding to 10 to 30 megabases) were cut in the dehydrated state using a dissection pipette mounted on a Leitz micromanipulator at a 45°C angle to the glass slide. Cutting of the fragments was achieved by lowering the dissection pipette and making contact with the glass slide at the approximate position of the chromosome region to be cut. Once contact was made, the downward motion due to the pliability of the glass pipette tip was translated into a sweep across the chromosome, thereby generating a fragment displaced from the remainder of the chromosome.

Pick Up and Injection of Chromosome Fragments:

The dissected fragments were then covered with a droplet of trypsin containing solution (0.05% trypsin/0.5 mM EDTA/0.1% polyvinylpyrrolidone/0.005% basic Fuchsin). At this point the dissection pipette was replaced by a holding pipette, and an injection pipette preloaded with silicone oil was mounted on a second micromanipulator. The tips of the injection and holding pipettes were dipped into the droplet of trypsin solution and the tip of the injection pipette was tapped against the holding pipette to achieve a final size of 0.5 to 1.0 um. Subsequently air and oil were ejected out of the injection pipette and a small volume of the trypsin solution was aspirated into the tip of the pipette. Next, the injection pipette was manipulated to dislodge the dissected fragment. This involved using the injection pipette to gently touch and push the dissected fragment so that it would detach from the glass slide and float. Then the fragment was aspirated into the pipette, and a small volume of 1% bovine serum albumin (BSA) was loaded into the tip to rinse the fragment.

For injection, the pipette preloaded with the chromosome fragment and BSA rinse solution was inserted with a single thrust into the pronucleus of a mouse egg immobilized by the holding pipette in a glass depression slide filled with Hepes-buffered Whitten's medium (See Whitten, W.K. Adv. Biosci 6:129, 1971). The liquid containing the chromosome fragment was then ejected and the pipette was withdrawn in one movement. The volume of liquid ejected is approximately 3 picoliters (i.e., the pronucleus increases in size by approximately 30 to 40%). Injected eggs which survived the micromanipulation were then collected and cultured in Whitten's medium to monitor their development.

[Selection of chromosomes to be dissected and the dissection and injection of chromosome fragments into mouse eggs were performed on a fixed-stage Leitz Diavert microscope using Leitz micromanipulators.

Fertilized eggs were obtained from oviducts excised from superovulated SWR/J females on the day of vaginal plug detection (following mating with SJL/J males), according to the procedures of Gordon, J.W. and Ruddle, F.H. "Gene Transfer into Mouse Embryos: Production -- Transgenic Mice by Pronuclear Injection", Methods in Enzymology, Vol. 101, pp. 411-433, Academic Press, 1983 and Hogan et al., Manipulating the Mouse Embryo, A Laboratory Manual, Cold Spring Harbor Laboratory, Cold Spring Harbor, New York, 1986. The eggs were injected with the chromosome fragment of the donor organism by the methods herein. After microinjection, the eggs were cultured for five days to monitor their development. Table 1 shows integration and maintenance of human chromosome fragments in the mouse embryos. Of 33 control embryos (injected with 50 mM KCl or the typsin solution), 21 (63%) survived the injection and 12 (57%) developed to the blastocyst stage (Table 1). For experimental embryos, 39 out of 90 (43%) survived the injection of centromeric fragments and 16 (41%) developed into blastocysts. Similarly, 17 out of 32 (53%) survived the injection of random chromosome fragments (fragments derived from non-centromeric regions) and 7 (41%) developed into blastocysts (Table 1). The remainder of the embryos (43-59%) became arrested at the 2- or 4-cell stage. Overall, with this injection technique 20% of the injected eggs developed to the blastocyst stage (Table 1).

Table 1

| Integration and Maintenance of Human Chromosome Fragments in Mouse Embryos | | | | |
|---|---|---|---|---|
| | | | Number of Embryos | |
| Fragment injected | Number of experiments | Survived/Injected[a] | Developed/Survived[b] | Fragment detection |
| Control | 2 | 21/33(63%) | 12/21(57%) | N.A. |
| Centromere | 6 | 39/90(43%) | 16/39(41%) | 6/12[c] |
| Random | 3 | 17/32(53%) | 7/17(41%) | 0/3[d] |
| NA Not Applicable | | | | |

a. Survived refers to the number of eggs still viable 2-4 hrs. after injection.
b. Developed refers to the number of embryos reaching the morula stage after 4 days in culture
c. In these six positive embryos, 16/82 nuclei and 11/89 metaphase spreads exhibited satellite DNA localization.
d. Also see text.

Incorporation of Human Chromosome Fragment Into Embryos:

To determine whether the human chromosome fragment was maintained in the developing embryo, experimental and control embryos that had developed to the morula stage by day 4 were incubated in Whitten's medium (Whitten, W.K., Adv. Biosci. 6: 129, 1971) containing 0.2 ug/ml colcemid. After 18 to 20 hr, embryos were transferred to a solution of 1:2 Whitten's medium/water for 3-5 min, then spread out on glass slides in cold 3:1 methanol/acetic acid [modified from Garside and Hillman, Experientia 41:1183, (1985)]. The presence of human chromosome fragments was detected by in situ hybridization using biotinylated nick translated DNA probes, according to the method of Lo, C.W., J. Cell Sci. 81: 143 (1986), in conjunction with a streptavidin-alkaline phosphatase (Enzo, Inc.) or a streptavidin-peroxidase/silver enhancement (Amersham Corp) detection scheme.

For the detection of injected centromeric fragments, the probe consisted of the plasmid pXBA-21 [provided by Dr. James E. Sylvester, University of Pennsylvania see, Thompson et al., Nucleic Acids Res. 17:2769-2782 (1989)] containing human satellite DNA inserts, and for the detection of random human chromosome fragments, plasmid pBLUR8 (Amersham, Corp.), which contains the human Alu I repeat was used. The in situ hybridization was carried out according to the method of Lo supra, except that no prehybridization treatments were necessary other than RNase A digestion. Detection using the peroxidase/silver method was performed using 30-50 sec incubation in diaminobenzidine and 6-8 min incubation in the silver amplification mix.

For three embryos injected with the random fragment, no hybridization signal was observed. However, given that the Alu I repeats are not homogeneously dispersed in the human genome, it is likely that a human chromosome fragment could have been missed due to the presence of few or no Alu I repeasts in conjunction with the limited detection sensitivity of the in situ procedure. With the satellite DNA probe, hybridization signals were observed within nuclei and also over individual chromosomes in 6 of 12 embryos. Hybridization signals were observed within nuclei and also detected in 4 of 13 injected embryos that had arrested at the 2-cell/4-cell stage of development (with signals observed only in one nucleus). The pattern of signal localization revealed that the injected fragments were very closely associated with individual chromosomes, thereby, suggesting that the exogenous human centromeric fragment was incorporated into the mouse karyotype. Note that as centromeric satellite DNA are organized as larger clusters of high copy tandem repeats as compared to the dispersed nature of the AluI repeats, the presence of a human centromeric fragment is expected to be much more easily detected as compared to a random human chromosome fragment.

Example 2

Improved Egg Survival With the Coinjection of Protease Inhibitors:

The methods of the invention were performed as in Example 1. The mobilizing solution was as described in Example 1, but the trypsin concentration was 0.025%. The physiologically acceptable liquid was modified to contain the protease inhibitors epsilon-amino caproic acid (50 mM) and benzamidine-HCl (5 mM). As shown in Table 2, the survival level was 66%. Thus, it was found that the inclusion of the two protease inhibitors resulted in more reproducible and higher efficiency of survival from the chromosome injection procedure.

Although the rate of development to the morula/blastocyst stage appears much lower (24% in Table 2 compared to 41% in Table 1), it was found after a large number of experiments, that the absence of the inhibitors, resulted in a highly variable rate of development. In contrast, the inclusion of inhibitors provided a very consistent rate of about 24% of the surviving embryos developing to the morula/blastocyst stage.

Table 2

| Egg Survival and Development With the Coinjection of Protease Inhibitors[a] | | | |
|---|---|---|---|
| Eggs Injected | | Survival (%) | Development[b] (%) |
| Experiments 1-6 | 71 | 51(72) | 11(21) |
| Experiments 7-12 | 68 | 41(60) | 10(24) |
| Experiments 13-18 | 88 | 57(65) | 15(26) |
| TOTAL | 227 | 149(66) | 36(24) |

a. Epsilon-amino caproic acid (50 mM) and Benzamidine-HCl(5mM)
b. To the morula/blastocyst stage.

Example 3

Postimplantion Development of Injected Eggs: (use of pseudopregnant females v. pregnant females):

The methods of the invention were performed as in Example 2. After microinjection of chromosome fragments, eggs were cultured in vitro to the blastocyst stage, then transferred in utero for further postimplantation development.

Pseudopregnant females:

In the first set of experiments, transfers were to CD-1 female mice mated with vasectomized SJL/J male mice (i.e. pseudopregnant females) and harvested on 8.5 days of gestation. Such transfers resulted in 6% of the eggs giving rise to embryos. This low yield probably resulted not from the inability of the embryos to develop normally, but from the very small number of embryos transferred to each surrogate female (see Table 3), and possibly the greater fragility of such implanted eggs.

Pregnant females:

To improve the efficiency with which the transferred eggs could develop further in vivo, in the second set of experiments, blastocyst embryos from chromosome fragment injected eggs were transferred to CD-1 females mated to normal B6D2F1/J males (i.e., pregnant females). Such females contained fertilized eggs, and therefore a set of developing embryos of their own. This fact should assist in the further development of the transferred eggs.

The presence of the uninjected normal embryos should facilitate the preparation of the uterine epithelium for implantation. This effect would result from cellular changes arising from hormone secretions induced as a consequence of the embryo-uterine interactions. In addition, the copresence of the uninjected eggs should provide a more hospitable microenvironment, perhaps via crossfeeding, for the further development of the injected eggs.

Embryos or mice developing from the transferred eggs can be distinguished from the embryos or mice of the recipient female on the basis of differences in eye pigmentation in embryos, or differences in eye pigmentation and coat color in mice, as the transferred embryos are albino while the embryos of the surrogate mother are pigmented (black eyes/agouti or black coat color). The transferred embryos were either collected on 12.5 days gestation or allowed to develop to term. In the experiment in which transferred embryos were collected on 12.5 days of development, 40% of the transferred blastocysts derived from chromosome-injected eggs further developed to give rise to normal 12.5 day embryos (see Table 4A). These results show a significant improvement in efficiency as compared to the 6% observed with embryos transferred to surrogate pseudopregnant females.

To determine the efficiency of life births from such chromosome fragment injected embryos, in another series of experiments, the embryos were either transferred in utero or in the oviduct of a pregnant female for further development to term.

In the experiments in which the chromosome-injected embryos were cultured to the morula/blastocyst stage and then transferred to the uterus of pregnant surrogate females for development to term, 15% of the transferred embryos gave rise to mice (see Table 4.B.2).

To examine if better efficiencies might be obtained by reducing the in vitro culture period of the injected mouse embryos, in a second set of experiments the chromosome injected embryos were transferred into the oviducts of pregnant CD1 females on day two, rather then in the uterus on day five of development. We observed that 26% of the injected eggs gave rise to live births (see Table 4.B.1). These results revealed a significant increase in the number of live pups that are delivered from the chromosome fragment injected eggs when the embryos were transferred into the oviduct of a pregnant female on day 2 of development.

Overall, these experiments show that eggs injected with chromosome fragments using the methods of the invention can undergo normal postimplantation development, and can result in mice being born. Hence the methods do not prevent the normal development of embryos to term.

It is also clear that the use of pregnant females rather than pseudopregnant females is essential in obtaining a reasonable efficiency of live births from the chromosome fragment injected eggs, and preferably such embryo transfers should be carried out into the oviduct.

Table 3.

| Postimplantation Development of Injected Eggs Transferred to Pseudopregnant Surrogate Mothers | | | | |
|---|---|---|---|---|
| Exp. No. | No. Embryo Transferred | # Surrogate Mother | Embryo[a] Recovered | Efficiency |
| 1 | 3 | 1 | 0 | 0% |
| 2 | 6 | 1 | 1 | 16% |
| 3 | 3 | 1 | 0 | 0% |
| 4 | 2 | 1 | 0 | 0% |
| 5 | 2 | 1 | 0 | 0% |
| TOTAL | 16 | 5 | 1 | 6% |

a. Embryos were harvested at 8.5 days gestation.

Table 4.

| Recovery of Embryos and Pups from Chromosome Injected Eggs Using Pregnant Females (Surrogate Mothers) | | | | | |
|---|---|---|---|---|---|
| A. Recovery of 12.5 Day Embryos | | | | | |
| Uterine Transfer[a] | Embryos Transferred | # Surrogate Mother | Inj-Embryos Recovered | CD1-Embryos[b] Recovered | Efficiency |
| Uninjected | 13 | 4 | 5 | 41 | 38% |
| Mock-Injected[c] | 11 | 3 | 5 | 27 | 45% |
| Chromosome-Injected | 20 | 11 | 8 | 73 | 40% |
| B. Recovery of Live Pups | | | | | |
|  | Embryos Transferred | # Surrogate Mother | Pups Born Inj Embryos | Pups Born CD1 Embryos[b] | Efficiency |
| 1. Oviduct Transfer | | | | | |
| Uninjected | 222 | 41 | 98 | 314 | 44% |
| Mock-Injected[c] | 152 | 31 | 61 | 219 | 40% |
| Chromosome-Injected | 135 | 41 | 36 | 343 | 26% |
| 2. Uterine Transfer[a] | | | | | |
| Uninjected | 97 | 18 | 38 | 113 | 39% |
| Chromosome-Injected | 13 | 7 | 2 | 56 | 15% |

a. Embryos were cultured to the morula-blastocyst stage and then transferred in utero for further development.

b. The CD1 fetuses/pups were distinguishable from those derived from the exogenously introduced embryos based on differences in coat color and eye pigmentation; the exogenous embryos are of albino genetic constitution, while the CD offspring are agouti or black as a result of mating with B6D2F1/J males.

c. Mock injection corresponds to the injection of 50 mM KCl or 50 mM epsilon amino caproic acid and 5 mM benzmidine-HCl in 10 mM Tris/0.1 mM EDTA (pH 7.5).

Example 4

Incorporation of Injected Fragment in the Postimplantation Embryo

An 8.5 day old embryo was fixed, embedded in paraffin, sectioned, and processed for in situ hybridization as in Example 1. In situ hybridization with biotinylated human satellite DNA probe showed that the human centromeric DNA was present in a cluster of cells in the embryo. This result suggests that the injected human centromeric fragment was incorporated in the mouse genome, and can be replicated and stably segregated through successive cell divisions.

An additional set of experiments was performed in which blastocysts developing from eggs after microinjection of a chromosome fragment were transferred to CD-1 surrogate mice and monitored for further post-implantation development. A second group of blastocysts developing from uninjected eggs or eggs injected with 50 mM KCl were similarly transferred and served as a control group. On day 13 of gestation, the embryos were recovered, fixed, embedded in paraffin, then processed for in situ hybridization as in Example 1. The results obtained are presented in Table 5.

Table 5

| Maintenance of Injected Human Chromosomal Fragments in Post-implantation Mouse Embryos | | | | | |
|---|---|---|---|---|---|
| | | Number of Embryos | | | |
| Fragment injected. | No. of experiments | Survived/Injected[a] | Developed/Survived[b] | Embryos Recovered[c] | Fragment detection |
| Control | 2 | 14/30(46%) | 9/14(64%) | 4(44%) | N.A. |
| Centromere | 10 | 74/135(54%) | 20/74(27%) | 8(40%) | 4/8 |
| NA Not Applicable | | | | | |

a. Survived refers to the number of eggs still viable 2-4 hrs. after injection.
b. Developed refers to the number of embryos reaching the morula stage after 4 days in culture.
c. 12.5 day embryos recovered from surrogate mothers.

As shown in Table 5, 4 out of 8 embryos injected with a human chromosome fragment positively showed the presence of human satellite DNA after in situ hybridization. These 4 positive embryos showed distinct clusters of cells with a strong hybridization signal over 20 to 90 consecutive sections, thus indicating the presence of a large number of cells containing human satellite DNA. In each positive embryo, at least 100 to 1000 cells were observed to contain the human satellite DNA. In contrast, no human satellite DNA was detected in any control embryos which were either not injected or injected with 50 mM KCl (i.e. no hybridization signal was observed in in situ hybridization).

Because only one centromeric fragment was injected into each embryo, these in situ hybridization results suggest that the exogenous human DNA underwent multiple rounds of replication in conjunction with mitotic cell divisions in the transomic animals.

Example 5

The methods of the invention were performed as in Example 2, except that a noncentromeric (i.e. random) human chromosome fragment was injected into mouse oocytes and the embryos were allowed to develop to term. Of 6 microinjected oocytes, 3 were transferred into surrogate CD-1 female mice. One live mouse was born. Analysis of DNA from tail tissue using conventional Southern blot hybridization revealed the presence of human Alu repeats (Figure 1), a family of dispersed repetitive DNA.

As demonstrated in Figure 1, MboI digested DNA from the mouse developed from a microinjected egg exhibited four distinct bands which are homologous to the human Alu repetitive DNA, thereby indicating the retention of human DNA (Figure 1, Lane 1). In contrast, MboI digested DNA from a normal or control mouse showed no bands, i.e., no human DNA (Figure 1, Lane 3). Thus, Figure 1 demonstrates that at least a portion of the random human chromosome fragment, microinjected according to the method of the invention, was retained. In summary, the results obtained clearly demonstrate that human chromosome fragments introduced into animal eggs, as described, can be propagated through multiple rounds of cell division, without which the human DNA could not have been detected in the tail tissue.

Obviously, numerous modifications and variations of the present invention are possible in light of the above teachings. It is therefore to be understood that within the scope of the appended claims, the invention may be practiced otherwise than as specifically described herein.

## Claims

1. A transgenic non-human organism, wherein said organism is an animal or a microbe, comprising at least one exogenous chromosome or at least one exogenous chromosome fragment in its heritable genetic material, wherein said exogenous chromosome and said exogenous chromosome fragment each contains at least 100 kilobases of nucleic acids.

2. An organism according to claim 1, wherein said exogenous chromosome or said exogenous chromosome fragment contains at least 250 kilobases of nucleic acids.

3. An organism according to claim 2, wherein said exogenous chromosome or said exogenous chromosome fragment contains at least 1 megabases of nucleic acids.

4. An organism according to claim 3, wherein said exogenous chromosome or said exogenous chromosome fragment contains from 10 to 30 megabases of nucleic acids.

5. An organism according to any one of the preceding claims, wherein said organism is a mammal.

6. An organism according to any one of the preceding claims, wherein said exogenous chromosome and said exogenous chromosome fragment is of animal, plant or microbial origin.

7. An organism according to any one of the preceding claims, wherein said exogenous chromosome or said exogenous chromosome fragment comprises genetic material which imparts to said organism characteristics useful for modeling pathological conditions arising from infectious agents or environmental agents.

8. An organism according to claim 7, wherein said infectious agent is an AIDS virus or a hepatitis virus.

9. A transgenic animal cell comprising at least one exogenous chromosome or at least one exogenous chromosome fragment in its heritable genetic material, wherein said exogenous chromosome and said exogenous chromosome fragment contains at least 100 kilobases of nucleic acids.

10. A cell according to claim 9, wherein said cell is a pluripotential embryonic stem cell.

11. A transgenic animal cell obtained from a transgenic multicellular animal, said multicellular animal comprising a plurality of cells containing at least one exogenous chromosome or at least one exogenous chromosome fragment, each containing at least 100 kilobases of nucleic acids.

12. A cell according to claim 11, wherein said exogenous chromosome or said exogenous chromosome fragment comprises genetic material encoding (i) a human immunoglobulin complex, or a portion thereof, (ii) the human histocompatibility complex, or a portion thereof, (iii) a hormone, a growth factor, a clotting factor or an enzyme, (iv) a heritable disease in humans or (v) an infectious disease.

13. A transgenic organism, wherein said organism is a plant, comprising at least one selected exogenous chromosome or at least one selectd exogenous chromosome fragment in its heritable genetic material, wherein said exogenous chromosome and said exogenous chromosome fragment each contains at least 100 kilobases of nucleic acids and is of plant origin.

14. A transgenic organism, wherein said organism is a plant, comprising at least one exogenous chromosome or at least one exogenous chromosome fragment in its heritable genetic material, wherein said exogenous chromosome and said exogenous chromosome fragment each contains at least 100 kilobases of nucleic acids and is of animal or microbial origin.

15. A transgenic plant cell, comprising at least one selected exogenous chromosome or at least one selected exogenous chromosome fragment in its heritable genetic material, wherein said exogenous chromosome and said exogenous chromosome fragment each contains at least 100 kilobases of nucleic acids and is of plant origin.

16. A transgenic plant cell, comprising at least one exogenous chromosome or at least one exogenous chromosome fragment in its heritable genetic material, wherein said exogenous chromosome and said exogenous chromosome fragment each contains at least 100 kilobases of nucleic acids and is of animal or microbial origin.

17. A method for transferring at least one chromosome or chromosome fragment from a donor organism to a recipient organism, comprising:

(a) isolating a selected chromosome or a selected chromosome fragment of a donor organism; and

(b) inserting at least a portion of said selected chromosome or of said selected chromosome fragment of said donor organism into the heritable genetic material of a recipient organism.

18. A method according to claim 17, wherein step (b) comprises inserting said chromosome or said chromosome fragment into a cell of said recipient organism for incorporation into the heritable genetic material of said recipient organism.

19. A method according to claim 18, wherein said chromosome or said chromosome fragment is inserted into the cytoplasm, pronucleus or nucleus of a cell of said recipient organism.

20. A method according to any one of claims 17 to 19, wherein said recipient organism is a non-human mammal.

21. A method according to any one of claims 17 to 19, wherein said recipient organism is an animal cell or plant cell.

22. A method according to claim 21, wherein said animal cell is a rodent cell or an oocyte.

23. A method according to any one of claims 17 to 22, wherein step (a) comprises:

(a') preparing the chromosome or chromosomes of said donor organism for retrieval of said chromosome or of said chromosome fragment;

(b') selecting a chromosome or chromosome fragment to be retrieved;

(c') retrieving said chromosome or said chromosome fragment; and

(d') manipulating said retrieved chromosome or said chromosome fragment into a pipette, a microelectrode or a syringe, or encapsulating said chromosome or said chromosome fragment into a vesicle, or loading said chromosomme or said chromosome fragment onto a microprojectile.

24. A method according to claim 23, wherein step (a') comprises spreading said chromosome or said chromosomes in a fixative solution onto a surface and said fixative solution is subsequently removed.

25. A method according to any one of claims 17 to 24 wherein said chromosome or chromosome fragment which is inserted into the heritable genetic material of said recipient organism comprises genetic material encoding a human immunoglobulin commplex or portion thereof or a human histocompatability complex or portion thereof.

26. A method according to any one of claims 17 to 24, wherein said chromosome(s) or chromosome fragment(s) which is (are) inserted into the heritable genetic material of said recipient organism comprise(s) genetic material encoding a hormone, a growth factor, a clotting factor or an enzyme.

27. A method accoridng to any one of claims 17 to 24, wherein said chromosome(s) or chromosome fragment(s) which is inserted into the heritable genetic material of said recipient organism is a human chromosome or chromosome frragment.

28. A method according to any one of claims 17 to 24, wherein said chromosome(s) or chromosome fragment(s) which is inserted into the heritable genetic material of said recipient organism comprises human genetic material associated with a disease in humans.

29. A method according to claim 28, wherein said disease in humans is selected from Huntington's disease, Alzheimer's disease, adult polycystic kidney disease, Down's syndrome, polysyndactyly autoimmune, cardiovascular and metabolic diseases.

30. A method according to any one of claims 17 to 24, wherien said chromosome(s) or chromosome fragment(s) which is inserted into the heritable genetic material of said recipient organism comprises human genetic material associated with an enhanced risk of developing cutaneous malignant melanoma or other cancers.

31. A transgenic cell containing at least one exogenous chromosome or exogenous chromosome fragment introduced into said transgenic cell, or an antecedent generation of said transgenic cell, by a method for transferring a chromosome or chromosome fragment from a donor organism to a recipient cell, which method comprises:

(a) isolating a chromosome or chromosome fragment of a donor organism; and

(b) inserting a portion, comprising more than 100 kilobases of nucleic acids, of said chromosome or of said chromosome fragment of said donor organism into the heritable genetic material of a recipient cell (b1) thereby obtaining said transgenic cell or (b2) obtaining a transgenic animal from said recipient cell and subsequently obtaining said transgenic cell from said transgenic animal.

32. A non-human transgenic animal whose germ cells and somatic cells contain at least one exogenous chromosome or exogenous chromosome fragment introduced into an embryonic stage of said animal, or an ancestor of said animal, by a method for transferring a chromosome or chromosome fragment from a donor organism to a recipient organism, which method comprises:

(a) isolating a chromosome or chromosome fragment of a donor organism;

(b) inserting a portion, comprising more than 100 kilobases of nucleic acids, of said chromosome or said chromosome fragment of said donor organism into the heritable genetic material of a recipient cell; and

(c) obtaining said non-human transgenic animal from said recipient cell.

33. An animal according to claim 32, which is a non-human mammal.

34. An animal according to claim 33, wherein said non-human mammal is a mouse.

35. A method for obtaining a transgenic animal having a plurality of cells containing at least one exogenous chromosome or exogenous chromosome fragment, said method comprising:

(a‴) introducing at least one selected exogenous chromosome or at least one selected exogenous chromosome fragment into the heritable genetic material of an embryonic animal recipient cell;

(b‴) transplanting said embryonic animal recipient cell containing said exogenous chromosome or said exogenous chromosome fragment into the oviduct or uterus of a female; and

(c‴) allowing said embryonic animal recipient cell to develop to term to obtain said transgenic animal.

36. A method for obtaining a transgenic animal having a plurality of cells containing at least one exogenous chromosome or exogenous chromosome fragment, said method comprising:

(a) introducing at least one exogenous chromosome or exogenous chromosome fragment, each containing at least 100 kilobases of nucleic acids, into the heritable genetic material of an embryonic animal receipient cell;

(b) generating a pluripotential embryonic stem cell line from said embryonic animal recipient cell;

(c) transplanting said pluripotential embryonic stem cell line into a developing embryo to make a chimeric embryo; and

(d) allowing said chimeric embryo to develop to term.

37. A method for obtaining a transgenic animal having a plurality of cells containing at least one exogenous chromosome or exogenous chromosome fragment, said method comprising:

(a) introducing at least one exogeneous chromosome or exogenous chromosome fragment, each containing at least 100 kilobases of nucleic acids, into the heritable genetic material of a pluripotential embryonic stem cell;

(b) transplanting said pluripotential embryonic stem cell line into a developing embryo to make a chimeric embryo; and

(c) allowing said chimeric embryo to develop to term.

38. A method for producing a human monoclonal antibody against an antigen, comprising:

harvesting antibodies from a hybridoma cell line formed by fusing, with a myeloma cell, a B cell lymphocyte capable of producing a human antibody against an antigen, wherein said B cell lymphocyte is obtained from a transgenic animal;

where said transgenic animal is an animal which comprises at least some cells containing at least one exogenous chromosome or exogenous chromosome fragment, each comprising more than 100 kilobases of nucleic acids.

39. A method for producing a hybridoma cell line which produces a human monoclonal antibody against an antigen, comprising:

(a″) immunizing a transgenic animal comprising at least some cells which contain at least one exogenous chromosome or exogenous chromosome fragment, each comprising more than 100 kilobases of nucleic acids;

(b″) harvesting B cell lymphocytes capable of producing antibody against said antigen from the lymph node, spleen or peripheral blood of said immunized transgenic animal; and

(c″) fusing said harvested B cell lymphocytes with a myeloma cell line to produce a hybridoma cell line which produces antibodies against said antigen.

40. A method according to claim 38 or 39, in which said transgenic animal is a mouse and said myeloma cell is a murine myeloma cell or said transgenic animal is a rat and said myeloma cell is a murine or rat myeloma cell.

41. A monoclonal antibody produced by a method comprising: harvesting antibodies from a hybridoma cell line formed by fusing, with a myeloma cell, a B cell lymphocyte capable of producing an antibody against an antigen, wherein said B cell lymphocyte is obtained from a transgenic animal comprising at least some cells which contain an exogenous chromosome or exogenous chromosome fragment, each comprising more than 100 kilobases of nucleic acids.

42. A hybridoma cell line produced by a method comprising:

(a‴) immunizing, with an antigen, a transgenic animal comprising at least some cells which contain at least one exogenous chromosome or exogenous chromosome fragment, each comprising more than 100 kilobases of nucleic acids, and comprising genetic material encoding a human immunoglobulin complex or a portion thereof;

(b‴) harvesting B cell lymphocytes capable of producing antibody against said antigen from the lymph node, spleen or peripheral blood of said immunized transgenic animal; and

(c‴) fusing said harvested B cell lymphocytes with a myeloma cell line to produce a hybridoma cell line which produces antibodies against said antigen.

# FIG. 1

**1 2 3**

1.8—

1.3—
1.1—
0.9—